# EUROPÄISCHE PATENTSCHRIFT

(11) **EP 3 352 817 B1**
(45) Veröffentlichungstag und Bekanntmachung des Hinweises auf die Patenterteilung: **24.03.2021**
(21) Anmeldenummer: 16774472.1
(22) Anmeldetag: 22.09.2016
(51) Int. Cl.: A61M 5/20

(54) **ALS AUTOINJEKTOR AUSGEBILDETE INJEKTIONSVORRICHTUNG**
INJECTION DEVICE, IN PARTICULAR AN AUTO-INJECTOR
DISPOSITIF D'INJECTION, NOTAMMENT AUTO-INJECTEUR

(30) Priorität: 24.09.2015 AT 508142015
(43) Veröffentlichungstag der Anmeldung: 01.08.2018
(73) Patentinhaber: Pharma Consult Ges.m.b.H., 1210 Wien (AT)
(72) Erfinder: CSENAR, Markus, 1050 Wien (AT); SCHWIRTZ, Andreas, 1090 Wien (AT)
(74) Vertreter: Burger, Hannes
(86) Internationale Anmeldenummer: PCT/EP2016/072585
(87) Internationale Veröffentlichungsnummer: WO 2017/050919

(56) Entgegenhaltungen:
- WO-A1-2011/101378
- WO-A1-2012/085589
- DE-A1-102007 013 836
- US-A- 5 354 286

## Beschreibung

Die Erfindung betrifft eine Injektionsvorrichtung, welche als Autoinjektor zur automatisierten Abgabe einer Injektionsflüssigkeit ausgebildet ist.

Aus der WO 2017/009284 A ist eine gattungsgemäß ausgebildete Injektionsvorrichtung mit einer Sicherungsbaugruppe gegen die ungewollte Auslösung und damit verbundene Abgabe der abzugebenden Injektionsflüssigkeit bekannt geworden. Die Injektionsvorrichtung umfasst ein hülsenförmig ausgebildetes Basisgehäuse, eine Karpule mit der darin enthaltenen und beim Injektionsvorgang abzugebenden Injektionsflüssigkeit, eine Nadelanordnung und eine Antriebsbaugruppe mit einem Antriebsmittel zur automatischen Abgabe der abzugebenden Injektionsflüssigkeit beim Injektionsvorgang. Von der Sicherungsbaugruppe wird die Antriebsbaugruppe bis zu deren Aktivierung für den Injektionsvorgang bezüglich des Basisgehäuses in ihrer Position festlegt. Die Sicherungsbaugruppe umfasst eine Sicherungskappe sowie zumindest einen Haltearm, wobei die Sicherungskappe einen Kappenmantel, eine Kappenwand sowie einen innerhalb des Kappenmantels sowie in einem Zentrum an der Kappenwand angeordneten und sich in Richtung einer Längsachse erstreckenden Sicherungsstift umfasst. Zwischen der Sicherungskappe und dem Basisgehäuse ist eine Führungsanordnung mit zumindest einem ersten Führungselement und mit zumindest einem damit zusammenwirkenden zweiten Führungselement vorgesehen. Die Sicherungskappe ist am Basisgehäuse von ihrer Sicherungsstellung in Axialrichtung sowie auf die vom distalen Ende des Basisgehäuses abgewendete Seite in eine Freigabestellung verstellbar. Der axiale Verstellweg wird dabei vom ersten und zweiten Führungselement begrenzt. In der Freigabestellung ist die Sicherungskappe noch am Basisgehäuse angeordnet und gehalten, wobei weiters vom Sicherungsstift der zumindest eine Haltearm für die Auslösung und Aktivierung der Antriebsbaugruppe freigegeben ist. Dabei konnte zwar die Abnahme der Sicherungskappe vom Basisgehäuse sowie eine Fehlorientierung bei der Abgabe verhindert werden, jedoch konnte der bislang von den Benutzern eingeübte Abgabevorgang mit der ansonsten üblichen Abnahme eines kappenförmigen Bauteils nicht mehr durchgeführt werden.

Die DE 10 2007 013 836 A1 beschreibt eine weitere gattungsgemäß ausgebildete Injektionsvorrichtung, insbesondere einen Autoinjektor, mit einem gesteuerten Nadelrückzug. Die Injektionsvorrichtung ist von einer Aufbewahrungsstellung in eine Injektionsstellung verstellbar ausgebildet und umfasst ein hülsenförmig ausgebildetes Basisgehäuse, welches ein einem Patienten zuwendbares distales Ende und ein davon abgewendetes proximales Ende aufweist und sich zwischen den Enden eine Längsachse erstreckt. Weiters umfasst die Injektionsvorrichtung eine Karpule mit der darin enthaltenen und beim Injektionsvorgang abzugebenden Injektionsflüssigkeit sowie eine Nadelanordnung und eine Sicherungsbaugruppe. Die Nadelanordnung ist der Karpule in der Aufbewahrungsstellung der Injektionsvorrichtung im Abschnitt des distalen Endes vorgeordnet. Die Sicherungsbaugruppe ist im Bereich des proximalen Endes des Basisgehäuses angeordnet und hält die Antriebsbaugruppe bis zu deren Aktivierung für den Injektionsvorgang bezüglich des Basisgehäuses in Position. Die Sicherungsbaugruppe umfasst eine Sicherungskappe mit einem Kappenmantel, einer Kappenwand sowie einen innerhalb des Kappenmantels sowie in einem Zentrum an der Kappenwand angeordneten und sich in Richtung der Längsachse erstreckenden Sicherungsstift. Die Sicherungskappe ist in der Aufbewahrungsstellung am Basisgehäuse in einer Sicherungsstellung für die zumindest eine Antriebsbaugruppe gehalten, und der Sicherungsstift hält in der Sicherungsstellung zumindest einen Haltearm der Sicherungsbaugruppe in radialer Richtung bezüglich der Längsachse ortsfest positioniert. Der Haltearm bildet ein Verbindungsglied zur Antriebsbaugruppe. Weiters ist die Sicherungskappe zur Freigabe der Antriebsbaugruppe ausgehend von ihrer Sicherungsstellung in Umfangsrichtung um die Längsachse in eine Freigabestellung verstellbar. Dazu ist eine Führungsanordnung mit einem ersten Führungselement und mit einem damit zusammenwirkenden zweiten Führungselement vorgesehen. In der Freigabestellung ist die Sicherungskappe noch am Basisgehäuse angeordnet und gehalten, wobei weiters vom Sicherungsstift der Haltearm für die Auslösung und Aktivierung der Antriebsbaugruppe freigegeben ist.

Die WO 2012/085589 A1 beschreibt einen Autoinjektor mit einem hülsenförmig ausgebildeten Basisgehäuse, in welchem eine Karpule mit der darin enthaltenen und beim Injektionsvorgang abzugebenden Injektionsflüssigkeit, eine Nadelanordnung, eine Antriebsbaugruppe sowie eine Sicherungsbaugruppe angeordnet sind. Die Sicherungsbaugruppe legt die Position der Antriebsbaugruppe bis zu deren Aktivierung für den Injektionsvorgang bezüglich des Basisgehäuses fest, wobei diese eine Sicherungskappe mit einem Kappenmantel, einer Kappenwand sowie einen innerhalb des Kappenmantels sowie an der Kappenwand angeordneten und sich in Richtung der Längsachse erstreckenden Sicherungsstift umfasst. Der Sicherungsstift drückt in der Sicherungsstellung die Haltearme der Sicherungsbaugruppe in radialer Richtung nach außen und hält diese somit bezüglich der Längsachse ortsfest positioniert, wobei die Haltearme Verbindungsglieder zur Antriebsbaugruppe bilden. Die Sicherungskappe ist von ihrer Sicherungsstellung in Axialrichtung sowie auf die vom distalen Ende abgewendete Seite in eine Freigabestellung zu verlagern und dabei vollständig abzunehmen. Weiters umfasst die Sicherungsbaugruppe auch noch eine innerhalb der Sicherungskappe angeordnete und am Basisgehäuse gehaltene Auslösekappe. Die Auslösekappe weist in deren Zentrum eine Öffnung zur Aufnahme des Sicherungsstifts der Sicherungskappe auf. Die zentrale Öffnung in der Auslösekappe ist von einem rohrförmigen Ansatz umgeben, welcher bei der Axialverstellung der Auslösekappe in Richtung auf das distale Ende mit den Rastarmen in Kontakt kommt und diese in radialer Richtung sowie in Richtung auf die Längsachse nach innen verlagert und damit die verrastete Position löst und der automatisierte Abgabevorgang erfolgt. Die Auslösekappe ist in ihrer Ausgangslage in proximaler Richtung am Basisgehäuse relativ zu diesem ortsfest gehalten und es ist nur ein Verstellweg in distaler Richtung für die Durchführung der Auslösebewegung möglich. Die Rückstellung der Auslösekappe von ihrer Auslösestellung in ihre Ausgangslage erfolgt mittels von der Auslösekappe in Axialrichtung vorragenden Federarmen im Zusammenwirken mit an der Innenseite des Basisgehäuses geneigt verlaufenen Stellflächen.

Aus der WO 2011/101378 A1 sowie aus der dazu parallel laufenden US 2013/01900693 A1 ist ebenfalls eine gattungsgemäß ausgebildete Injektionsvorrichtung, insbesondere ein Autoinjektor, bekannt geworden. Auch diese Injektionsvorrichtung umfasst unter anderem eine im Bereich eines proximalen Endes befindliche Sicherungsbaugruppe für eine Antriebsbaugruppe. Die Sicherungsbaugruppe umfasst eine ausschließlich in Umfangsrichtung um eine Längsachse von einer Sicherungsstellung in eine Freigabestellung verschwenkbare Sicherungshülse. Dazu ist eine Führungsanordnung mit ersten Führungselementen und damit zusammenwirkenden zweiten Führungselementen vorgesehen. In der Freigabestellung ist die Sicherungshülse noch am Basisgehäuse angeordnet und gehalten, wobei in der Sicherungsstellung ein Auslöseknopf zur Auslösung der Antriebsbaugruppe von einem innenseitig an der Sicherungshülse befindlichen Vorsprung an einer Axialverstellung in Richtung auf das distale Ende der Injektionsvorrichtung gehindert ist. Ist durch die Drehbewegung der Sicherungshülse der Vorsprung wegverstellt worden, ist eine zuvor am Vorsprung anliegende längliche Rippe freigegeben und die Auslösung der Antriebsbaugruppe zur automatischen Abgabe des Medikaments kann mittels des Auslöseknopfs erfolgen.

Eine andere gattungsgemäß ausgebildete Injektionsvorrichtung, insbesondere einen Autoinjektor, beschreibt die US 5,354,286 A1. Die Injektionsvorrichtung ist von einer Aufbewahrungsstellung in eine Injektionsstellung verstellbar ausgebildet und umfasst ein hülsenförmig ausgebildetes Basisgehäuse, welches ein einem Patienten zuwendbares distales Ende und ein davon abgewendetes proximales Ende aufweist und sich zwischen den Enden eine Längsachse erstreckt. Deren Sicherungsbaugruppe ist ebenfalls im Bereich des proximalen Endes des Basisgehäuses angeordnet und hält die Antriebsbaugruppe bis zu deren Aktivierung für den Injektionsvorgang bezüglich des Basisgehäuses in Position. Die Sicherungsbaugruppe umfasst eine Sicherungskappe mit einem Kappenmantel, einer Kappenwand sowie einen innerhalb des Kappenmantels sowie in einem Zentrum an der Kappenwand angeordneten und sich in Richtung der Längsachse erstreckenden Sicherungsstift. Die Sicherungskappe ist in der Aufbewahrungsstellung am Basisgehäuse in einer Sicherungsstellung für die zumindest eine Antriebsbaugruppe gehalten. In der Sicherungsstellung hält der Sicherungsstift einen Haltearm der Sicherungsbaugruppe in radialer Richtung bezüglich der Längsachse ortsfest positioniert. Zur Freigabe und Auslösung der Antriebsbaugruppe ist die Sicherungskappe mitsamt dem darin angeordneten Sicherungsstift vom Basisgehäuse in Axialrichtung auf die vom distalen Ende abgewendete Richtung abzuziehen und vom Basisgehäuse zu entfernen. Damit verbleibt im Bereich des proximalen Endes des Basisgehäuses eine Öffnung sichtbar.

Aus der US 6,767,336 B1 ist eine automatisierte Injektionsvorrichtung zur Abgabe eines Medikaments bekannt geworden, die ein Traggehäuse umfasst, in dem die Antriebsbaugruppe, der Speicherbehälter für das Medikament sowie die Nadelanordnung angeordnet ist. Über die Kanüle der Nadelanordnung ist ein elastisch verformbares und durchstechbares Schutzelement übergestülpt, welches mit dem Nadelhalter verbunden ist. Der Nadelhalter trägt einerseits die Nadel und ist am Speicherbehälter für das Medikament an seinem distalen Ende angekuppelt. Die Kanüle steht dabei mit dem Innenraum und somit mit dem Medikament stets in Strömungsverbindung. Im Bereich des distalen Endes ist am Traggehäuse ein Nadelschutzelement längs verschieblich gelagert, welchem ein weiteres Antriebsmittel zugeordnet ist. Ausgehend vom proximalen Ende des Traggehäuses ist diesem eine Auslösehülse zugeordnet bzw. nimmt diese das Traggehäuse zumindest bereichsweise auf. Der ersten Antriebsbaugruppe ist weiters ein kappenförmiges Sicherungselement mit einem in die Injektionsvorrichtung hinein ragenden Sicherungsstift zugeordnet, um eine ungewollte Auslösung zu vermeiden. Zur Entsicherung ist die Kappe mitsamt dem Sicherungsstift vom Traggehäuse vollständig abzuziehen. Die Auslösung des Nadelschutzelements bzw. dessen Antriebsmittel erfolgt während der relativen Verlagerung des Medikamentenspeichers mit samt der daran angeordneten Nadelanordnung. Sowohl die Auslösehülse als auch das Nadelschutzelement sind jeweils eng anliegend sowie in axialer Richtung hintereinander am gemeinsamen Traggehäuse angeordnet. Nachteilig dabei ist, dass nach dem Abziehen der Sicherungskappe sowohl im Bereich des distalen Endes als auch im Bereich des proximalen Endes des Traggehäuses eine Öffnung sichtbar ist.

Die US 4,031,893 A beschreibt einen weiteren ähnlich ausgebildeten Autoinjektor, bei welchem in einem gemeinsamen Traggehäuse die auslösbare Antriebsbaugruppe und der Medikamentenbehälter mit der daran angeordneten Nadelanordnung aufgenommen ist. Die Antriebsbaugruppe ist wiederum durch ein kappenförmig ausgebildetes Sicherungselement mit einem in die Injektionsvorrichtung hinein ragenden Sicherungsstift gegen unbeabsichtigtes Auslösen gesichert. Das Traggehäuse ist an seiner Außenseite von einem hülsenförmigen Bauteil umgeben, welcher an seinem proximalen Ende mit einer zusätzlichen Auslösehülse gekuppelt ist. Die Auslösehülse bewirkt nach dem gänzlichen Entfernen der Sicherungskappe mitsamt dem Sicherungsstift die Auslösung der Antriebsbaugruppe, wodurch der Medikamentenbehälter mitsamt der Nadelanordnung in Richtung des distalen Endes verstellt wird. Die mit dem Innenraum des Medikamentenbehälters stets in Strömungsverbindung stehende Kanüle ist von einer elastisch verformbaren Schutzhülle umgeben, welche an der Innenseite des distalen Endes des Traggehäuses abgestützt ist. Während der Vorwärtsbewegung durchsticht die Kanüle die elastisch verformbare Schutzhülle und tritt aus dem Traggehäuse zur Injektion aus. Die elastisch verformbare Nadelschutzhülle bewirkt nach Beenden des Injektionsvorganges eine Rückstellung der Nadelanordnung mitsamt dem Medikamentenbehälter in den Innenraum des Traggehäuses. Auch hier ist nachteilig, dass nach dem Abziehen der Sicherungskappe sowohl im Bereich des distalen Endes als auch im Bereich des proximalen Endes des Traggehäuses eine Öffnung sichtbar ist.

Aus der US 5,295,965 A ist eine automatisierte Injektionsvorrichtung zur Abgabe eines Medikaments bekannt geworden, bei welcher die äußere Umhüllung eine Nadelschutzhülse darstellt. Diese reicht von dem dem Patienten zugewendeten distalen Ende bis nahe an das proximale Ende hin und kann dort mit einer kappenförmigen Auslösemuffe betätigt werden. Für die Aktivierung ist ein im Bereich des proximalen Endes angeordneter Sicherungsstift zu lösen, der die erste Antriebsbaugruppe bis zur Auslösung mit der kappenförmigen Auslösemuffe sichert. Nach dem Entfernen des Sicherungsstifts, kann die muffenförmige Auslösemuffe relativ gegenüber dem Traggehäuse sowie der Nadelschutzhülse bewegt werden, wodurch sowohl die erste Antriebsbaugruppe als auch die Nadelschutzhülse ausgelöst wird. Der Nadelschutzhülse ist ihrerseits eine eigene Federvorrichtung zugeordnet, mit welcher dieser in die die Nadel abdeckende Stellung verstellt wird. Auch hier ist nachteilig, dass nach dem Abziehen des Sicherungsstiftes sowohl im Bereich des distalen Endes als auch im Bereich des proximalen Endes des Traggehäuses eine Öffnung sichtbar ist.

Aus der US 5,658,259 A bzw. der EP 0 956 058 B1 ist eine weitere automatisierte Injektionsvorrichtung zur Abgabe eines Medikaments aus einer Karpule bekannt geworden. Dazu wird innerhalb eines Traggehäuses die Karpule mit dem Medikament angeordnet, wobei dieser zusätzlich noch eine erste Antriebsbaugruppe für deren Betätigung und die Abgabe des Medikaments zugeordnet ist. Innerhalb des Traggehäuses ist eine durch die Längsverstellung des Nadelträgers auslösbare Nadelschutzhülse angeordnet, welcher ebenfalls ein Verstellelement zugeordnet ist. Zur Freigabe aus der Sperrstellung ist zuerst im Bereich des proximalen Endes der Injektionsvorrichtung eine Abdeckkappe abzuziehen, wodurch ein Zugang zu einem Sicherungsstift ermöglicht wird. Der Sicherungsstift ist zur Betätigung der ersten Antriebsbaugruppe in Richtung auf das distale Ende einzudrücken. Die Nadelschutzhülse wird innerhalb des Tragkörpers mittels einer Rastverbindung bis zu deren Auslösung durch den verstellten Nadelträger gehalten. Nach der Abgabe des Medikaments deckt die Nadelschutzhülse das über den Autoinjektor vorragende Ende der Nadel ab. Diese Injektionsvorrichtung weist eine andere Auslösebaugruppe und Wirkungsweise auf, da die Injektionsvorrichtung zuerst auf die gewünschte Einstichstelle aufgesetzt werden muss und erst nachfolgend die Auslösung der ersten Antriebsbaugruppe durch das Hineindrücken des Sicherungsstifts erfolgt.

Die WO 2005/113039 A1 beschreibt einen anders ausgebildeten Autoinjektor zur Abgabe eines Medikaments, der ein feststehendes Traggehäuse mit einer inneren und äußeren Gehäusewand im Bereich des distalen Endes umfasst. Im Traggehäuse ist die Antriebsbaugruppe, der Speicherbehälter für das Medikament sowie die Nadelanordnung angeordnet. Das proximale Ende der Nadel steht mit dem Innenraum des Speicherbehälters und somit mit dem Medikament stets in Strömungsverbindung. Weiters umfasst die automatische Injektionsvorrichtung ein in Richtung der Längsachse verschiebbares Nadelschutzelement, welches zwischen der inneren und äußeren Gehäusewand des Traggehäuses geführt gelagert ist. Diesem hülsenförmigen Nadelschutzelement ist eine weitere Antriebsbaugruppe zugeordnet, um dieses nach der Freigabe in die abdeckende Stellung der Nadel zu verbringen. Zusätzlich kann an der Außenseite des Traggehäuses eine die Handhabbarkeit verbessernde Grifflage angeordnet sein. An dem der Nadel gegenüberliegenden Ende - also dem proximalen Ende - ist eine Auslöseeinrichtung zur Auslösung der ersten Antriebsbaugruppe angeordnet. Dabei erfolgt die Auslösebewegung durch eine Schiebebewegung in senkrechter Richtung bezüglich der Längsachse. Nach erfolgter Querverlagerung und der Entriegelung der Rückhaltevorrichtung wird die erste Antriebsbaugruppe aktiviert, wodurch der Kolben mitsamt dem Speicherbehälter soweit in Richtung des distalen Endes verlagert, bis dass ein dem proximalen Ende zugewendeter Anschlag des Speicherbehälters an einem Anschlag der inneren Gehäusewand des Traggehäuses zur Anlage kommt. Gleichzeitig mit dieser Längsverstellung erfolgt auch die Auslösung des Nadelschutzelements, welches dadurch bis hin zur Anlage an der Abgabestelle des Medikaments bewegt wird. Nach erfolgter Abgabe des Medikaments und dem Abziehen der gesamten Injektionsvorrichtung erfolgt aufgrund der bereits aktivierten weiteren Antriebsvorrichtung eine weitere Vorwärtsbewegung des Nadelschutzelements, bis dass die Nadel vollständig abgedeckt ist.

Aufgabe der vorliegenden Erfindung war es, die Nachteile des Standes der Technik zu überwinden und eine Injektionsvorrichtung zur Verfügung zu stellen, mittels derer ein Benutzer in der Lage ist, eine einfache und sichere Abgabe des Medikaments vornehmen zu können und dabei eine falsche Anwendungsrichtung und damit einhergehende mögliche ungewollte Stichverletzungen zu vermeiden.

Diese Aufgabe wird durch eine Injektionsvorrichtung gemäß den Ansprüchen gelöst.

Die erfindungsgemäße Injektionsvorrichtung ist von einer Aufbewahrungsstellung in eine Injektionsstellung verstellbar ausgebildet und umfasst
- ein hülsenförmig ausgebildetes Basisgehäuse, welches Basisgehäuse ein einem Patienten zuwendbares distales Ende und ein davon abgewendetes proximales Ende aufweist, zwischen welchen Enden sich eine Längsachse erstreckt,
- eine Karpule mit einer darin enthaltenen und beim Injektionsvorgang abzugebenden Inj ektionsflüssigkeit,
- eine Nadelanordnung, die der Karpule in der Aufbewahrungsstellung der Injektionsvorrichtung im Abschnitt des distalen Endes vorgeordnet ist
- zumindest eine Antriebsbaugruppe, welche Antriebsbaugruppe ein Antriebsmittel zur automatischen Abgabe der abzugebenden Injektionsflüssigkeit beim Injektionsvorgang umfasst,
- eine Sicherungsbaugruppe, welche Sicherungsbaugruppe die zumindest eine Antriebsbaugruppe bis zu deren Aktivierung für den Injektionsvorgang bezüglich des Basisgehäuses in ihrer Position festlegt,
- wobei die Sicherungsbaugruppe eine Sicherungskappe sowie zumindest einen Haltearm umfasst, wobei die Sicherungskappe einen Kappenmantel, eine Kappenwand sowie einen innerhalb des Kappenmantels sowie in einem Zentrum an der Kappenwand angeordneten und sich in Richtung der Längsachse erstreckenden Sicherungsstift umfasst,
- und wobei die Sicherungskappe in der Aufbewahrungsstellung außenseitig am Basisgehäuse in einer Sicherungsstellung für die zumindest eine Antriebsbaugruppe gehalten ist, und der Sicherungsstift in der Sicherungsstellung innerhalb des zumindest einen Haltearms im axialen Zentrum der Sicherungsbaugruppe angeordnet ist und den zumindest einen Haltearm der Sicherungsbaugruppe in radialer Richtung bezüglich der Längsachse an einer radialen Verstellung nach innen in Richtung auf die Längsachse ortsfest positioniert hält, und der zumindest eine Haltearm ein Verbindungsglied zur Antriebsbaugruppe bildet,
- wobei zwischen der Sicherungskappe und dem Basisgehäuse eine Führungsanordnung mit zumindest einem ersten Führungselement und mit zumindest einem damit zusammenwirkenden zweiten Führungselement vorgesehen ist,
- wobei die Karpule, die Nadelanordnung, die zumindest eine Antriebsbaugruppe sowie die Sicherungsbaugruppe zumindest bereichsweise im Basisgehäuse aufgenommen oder angeordnet sind, und die Sicherungsbaugruppe sowie die Antriebsbaugruppe in jenem Teil des Basisgehäuses angeordnet sind, welcher das proximale Ende ausbildet, und
- wobei das zumindest eine erste Führungselement durch einen Fortsatz gebildet ist, welcher an einer Innenfläche des Kappenmantels angeordnet oder ausgebildet ist und dieser in Richtung auf die Längsachse vorragt,
- wobei das zumindest eine zweite Führungselement durch eine vertieft im Basisgehäuse ausgebildete Führungsbahn gebildet ist, welche am Basisgehäuse im Bereich des proximalen Endes angeordnet oder ausgebildet ist, oder wobei das zumindest eine zweite Führungselement durch zumindest eine im Bereich des proximalen Endes des Basisgehäuses angeordnete oder ausgebildete, nutförmige Vertiefung und/oder einen Fortsatz gebildet ist,
- wobei die Sicherungskappe ausgehend von ihrer Sicherungsstellung in Axialrichtung sowie auf die vom distalen Ende abgewendete Seite in eine Freigabestellung verstellbar ist,
- wobei das zumindest eine erste Führungselement und das zumindest eine zweite Führungselement einen in Axialrichtung begrenzten Verstellweg der Sicherungskappe zwischen deren Sicherungsstellung und deren Freigabestellung festlegen, wodurch die Sicherungskappe in deren Freigabestellung noch am Basisgehäuse angeordnet und gehalten ist, und dabei weiters vom Sicherungsstift der zumindest eine Haltearm für die Auslösung und Aktivierung der Antriebsbaugruppe freigegeben ist, und dabei
- die Sicherungsbaugruppe weiters eine Betätigungskappe umfasst, welche lösbar an der Sicherungskappe gehalten ist,
- die Betätigungskappe die Sicherungskappe im Bereich ihres Kappenmantels übergreift, und
- zwischen der Betätigungskappe und der Sicherungskappe miteinander in Eingriff stehende Rastmittel vorgesehen sind.

Der dadurch erzielte Vorteil liegt darin, dass so eine mögliche Fehlorientierung der Injektionsvorrichtung nach der Entriegelung der Sicherungsbaugruppe und der damit verbundenen Freigabe für den Injektionsvorgang vermieden werden kann. Da die ansonsten abnehmbar am Basisgehäuse gehaltene Sicherungskappe mit ihrem Sicherungsstift in einen Durchbruch des Basisgehäuses hineinragt und damit die Haltearme positioniert hält, hinterlässt die Sicherungskappe nach deren Abnahme ein offenes Loch im Bereich der Rückwand des Basisgehäuses. So konnte es passieren, dass der Benutzer nicht das vorgesehene distale Ende, aus welchem die Kanüle für den Einstichvorgang hervortritt, der vorgesehenen Einstichstelle zugewendet hat, sondern das falsche proximale Ende, von welchem die Sicherungskappe vollständig abgezogen bzw. entfernt worden ist. Wird dabei auch z.B. noch der Daumen oder ein anderer Körperteil auf jenes Ende aufgelegt, welches nun der vorgesehenen Einstichstelle gegenüberliegt und aus welchem die Kanüle nach der Auslösung hervortritt, erfolgt der Einstich und damit verbunden die Abgabe der Injektionsflüssigkeit in diesen Körperteil und nicht in die dafür vorgesehene Einstichstelle. Da nun die Sicherungskappe auch noch nach dem Verstellen in die Freigabestellung am Basisgehäuse verbleibt und nicht mehr abgenommen werden kann, können derartige Fehlorientierungen ausgeschlossen werden. Diese mögliche Fehlorientierung kommt insbesondere bei Situationen vor, wo bereits eine erhöhte Stressbelastung für den Benutzer gegeben ist.

Ist das zumindest eine erste Führungselement durch einen Fortsatz gebildet, welcher an einer Innenfläche des Kappenmantels angeordnet oder ausgebildet ist und dieser in Richtung auf die Längsachse vorragt, kann dadurch eine sichere Verstellbewegung der Sicherungskappe am Basisgehäuse erzielt werden. Weiters werden damit aber auch störende Vorsprünge an der Sicherungskappe vermieden sowie eine umfänglich geschlossene Ausbildung des Kappenmantels erzielt werden.

Ist das zumindest eine zweite Führungselement durch eine im Basisgehäuse ausgebildete Führungsbahn gebildet, welche am Basisgehäuse im Bereich des proximalen Endes angeordnet oder ausgebildet ist, kann durch die Wahl der Führungsbahn der Längsverlauf und damit die Verstellbewegung der Sicherungskappe festgelegt werden. Darüber hinaus kann so aber auch eine eindeutige axiale Länge des Verstellwegs festgelegt werden. Dies auch in jenem Fall, wenn eine Schwenkung oder Drehbewegung der Sicherungskappe relativ bezüglich des Basisgehäuses gewählt ist. Ist hingegen das zumindest eine zweite Führungselement durch zumindest eine im Bereich des proximalen Endes des Basisgehäuses angeordnete oder ausgebildete bevorzugt umlaufende nutförmige Vertiefung und/oder einen Fortsatz gebildet, kann dadurch eine einfache Schnappverbindung zwischen der Sicherungskappe und dem Basisgehäuse in zumindest einer der Stellungen, nämlich der Sicherungsstellung und/oder der Freigabestellung, geschaffen werden.

Dadurch, dass die Sicherungsbaugruppe weiters eine Betätigungskappe umfasst, welche lösbar an der Sicherungskappe gehalten ist, kann so für den Benutzer bzw. Anwender die Möglichkeit geschaffen werden, nach dem Verstellen der Sicherungskappe in die Freigabestellung weiterhin ein kappenähnliches Bauteil von der Injektionsvorrichtung abnehmen zu können und trotzdem die Sicherungskappe weiterhin am Basisgehäuse gehalten verbleibt.

Es sind weiters zwischen der Betätigungskappe und der Sicherungskappe miteinander in Eingriff stehende Rastmittel vorgesehen. Damit kann auf einfache Art und Weise die Lösekraft der Betätigungskappe von der Sicherungskappe festgelegt werden.

Eine weitere mögliche Ausführungsform hat die Merkmale, dass die Führungsbahn in deren Längsverlauf bezüglich einer in senkrechter Richtung zur Längsachse ausgerichteten Ebene eine unterschiedliche Steigung mit zueinander unterschiedlichen Steigungswinkeln aufweist. Dadurch kann je nach gewähltem Steigungswinkel der relative Schwenk- bzw. Drehwinkel der Sicherungskappe relativ bezüglich des Basisgehäuses festgelegt werden.

Eine weitere Ausbildung sieht vor, dass der Steigungswinkel der Führungsbahn im Bereich der Sicherungsstellung und/oder der Freigabestellung flacher ausgebildet ist als in jenem sich zwischen der Sicherungsstellung und der Freigabestellung erstreckenden Zwischenabschnitt derselben. Dadurch kann in der Sicherungsstellung und/oder der Freigabestellung eine bessere axiale Positionierung der Sicherungskappe am Basisgehäuse erzielt werden.

Eine weitere bevorzugte Ausführungsform ist dadurch gekennzeichnet, dass die Sicherungsbaugruppe weiters eine Anzeigeeinheit umfasst, welche Anzeigeeinheit zumindest eine der beiden Stellungen der Sicherungskappe relativ bezüglich des Basisgehäuses anzeigt. Damit kann dem Benutzer mittels der Anzeigeeinheit auf einfache Art und Weise veranschaulicht werden, in welcher Stellung sich die Injektionsvorrichtung, insbesondere deren Sicherungsbaugruppe, befindet.

Weiters kann es vorteilhaft sein, wenn die Anzeigeeinheit zumindest ein erstes Anzeigemittel umfasst, welches im Bereich der Sicherungskappe angeordnet oder ausgebildet ist. Damit kann auf einfache Art und Weise die Sicherungskappe und deren relative Lage bezüglich des Basisgehäuses eindeutig dargestellt werden.

Eine andere alternative Ausführungsform zeichnet sich dadurch aus, dass das zumindest eine erste Anzeigemittel durch einen Durchbruch und/oder einen von der Kappenwand distanziert angeordneten Kappenrand des Kappenmantels gebildet ist. Damit wird die Möglichkeit geschaffen, auch bei einer zusätzlich zur axialen Verstellung erfolgten Dreh- bzw. Schwenkbewegung eine eindeutige Anzeige und damit Veranschaulichung des Betriebszustands der Injektionsvorrichtung erzielen zu können.

Eine weitere mögliche und gegebenenfalls alternative Ausführungsform hat die Merkmale, dass die Anzeigeeinheit zumindest ein zweites Anzeigemittel umfasst, welches im Bereich des Basisgehäuses angeordnet oder ausgebildet ist. Damit kann wiederum die relative Lage und Position der Sicherungskappe in zumindest einer relativen Stellung bezüglich des Basisgehäuses einfach dargestellt werden.

Eine weitere Ausbildung sieht vor, dass die Anzeigeeinheit weiters ein Anzeigeträgerelement umfasst, welches Anzeigeträgerelement innerhalb des Kappenmantels sowie unmittelbar benachbart zur Kappenwand angeordnet ist, und das Anzeigeträgerelement am Basisgehäuse in Axialrichtung verstellbar geführt ist und weiters die Sicherungskappe relativ bezüglich des Anzeigeträgerelements um die Längsachse verschwenkbar ist und weiters das Anzeigeträgerelement an der Sicherungskappe relativ bezüglich dieser in Richtung der Längsachse positioniert gehalten ist. Dadurch kann auch im Bereich der Kappenwand bzw. Stirnwand der Sicherungskappe auch bei einer relativen Verschwenk- bzw. Verdrehbewegung der Sicherungskappe bezüglich des Basisgehäuses stets eine eindeutige Betriebszustandsanzeige geschaffen werden. Damit kann das Anzeigeträgerelement ausschließlich in Axialrichtung bezüglich des Basisgehäuses an diesem verstellt werden und trotzdem eine Verschwenk- bzw. Drehbewegung der Sicherungskappe sowohl bezüglich des Anzeigeträgerelements als auch des Basisgehäuses durchgeführt werden.

Eine andere Ausführungsform zeichnet sich dadurch aus, dass das Anzeigeträgerelement einen scheibenförmig ausgebildeten Grundkörper mit einer in dessen Zentrum angeordneten Durchbrechung sowie zumindest einen in Axialrichtung davon abstehenden Führungssteg umfasst. Damit wird eine einfache Aufnahme des Anzeigeträgerelements innerhalb der Sicherungskappe ermöglicht und trotzdem eine sichere axiale Führung am Basisgehäuse erzielt. Eine weitere bevorzugte Ausführungsform ist dadurch gekennzeichnet, dass der zumindest eine Führungssteg im äußeren Umfangsbereich des scheibenförmig ausgebildeten Grundkörpers angeordnet ist. So kann mit geringstem Platzaufwand eine Aufnahme zwischen dem Kappenmantel und dem Basisgehäuse erzielt werden.

Weiters kann es vorteilhaft sein, wenn zwischen dem zumindest einen Führungssteg und dem Basisgehäuse eine Längsführungsanordnung angeordnet oder ausgebildet ist. Damit kann das gesamte Anzeigeträgerelement sicher und geradlinig am Basisgehäuse in Axialrichtung geführt werden.

Eine andere Ausführungsform zeichnet sich dadurch aus, dass die Anzeigeeinheit zumindest ein erstes Anzeigemittel umfasst, welches als Durchbruch in der Kappenwand der Sicherungskappe ausgebildet ist. Damit kann eine einfache optische Sichtbarkeit an der Injektionsvorrichtung geschaffen werden.

Eine weitere mögliche Ausführungsform hat die Merkmale, dass die Anzeigeeinheit zumindest ein zweites Anzeigemittel umfasst, welches am Anzeigeträgerelement angeordnet ist. Damit wird die Möglichkeit geschaffen, einen einfachen Wechsel der Darstellung der Anzeigemittel durchführen zu können. Dies kann beispielsweise durch einfachen Austausch des Anzeigeträgerelements erfolgen.

Eine weitere Ausbildung sieht vor, dass das zumindest eine zweite Anzeigemittel aus der Gruppe von Symbol, Farbkennung, Markierungsring, Bild, Blindenschrift, Brailleschrift ausgewählt ist. Damit kann auf unterschiedliche Einsatzanforderungen die jeweils dafür gewünschte, optische Darstellung gewählt werden.

Eine weitere mögliche Ausführungsform hat die Merkmale, dass an der Sicherungskappe als Rastmittel zumindest ein an einem Federarm angeordneter Rastfortsatz und an der Betätigungskappe als weiteres Rastmittel zumindest eine den Rastfortsatz aufnehmende Rastausnehmung vorgesehen sind.

Eine weitere bevorzugte Ausführungsform ist dadurch gekennzeichnet, dass die miteinander in Eingriff stehenden Rastmittel im Bereich des Kappenmantels vorgesehen sind.

Schließlich weist eine alternative Ausführungsform die Merkmale auf, dass die Betätigungskappe die Sicherungskappe vollständig in sich aufnimmt.

Zum besseren Verständnis der Erfindung wird diese anhand der nachfolgenden Figuren näher erläutert.

Es zeigen jeweils in stark vereinfachter, schematischer Darstellung:
- Fig. 1: eine mögliche Ausbildung einer Injektionsvorrichtung in deren Aufbewahrungsstellung, in schaubildlicher Darstellung;
- Fig. 2: eine mögliche Ausbildung einer Führungsanordnung an einer Sicherungskappe für die Injektionsvorrichtung, im Axialschnitt;
- Fig. 3: eine mögliche Ausbildung eines Basisgehäuses im Bereich von dessen proximalen Ende, für eine Sicherungskappe nach Fig. 2, in Ansicht;
- Fig. 4: eine weitere mögliche Ausbildung einer Sicherungsbaugruppe einer Injektionsvorrichtung, in schaubildlicher sowie voneinander distanzierter Darstellung der Bauteile;
- Fig. 5: eine weitere mögliche Ausbildung der Sicherungskappe mit einer darüber angeordneten, abnehmbaren Betätigungskappe, im Axialschnitt;
- Fig. 6: andere mögliche Ausbildungen der Sicherungsbaugruppe in noch voneinander distanzierter Darstellung der Bauteile, teilweise geschnitten.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

Der Begriff "insbesondere" wird nachfolgend so verstanden, dass es sich dabei um eine mögliche speziellere Ausbildung oder nähere Spezifizierung eines Gegenstands oder eines Verfahrensschritts handeln kann, aber nicht unbedingt eine zwingende, bevorzugte Ausführungsform desselben oder eine Vorgehensweise darstellen muss.

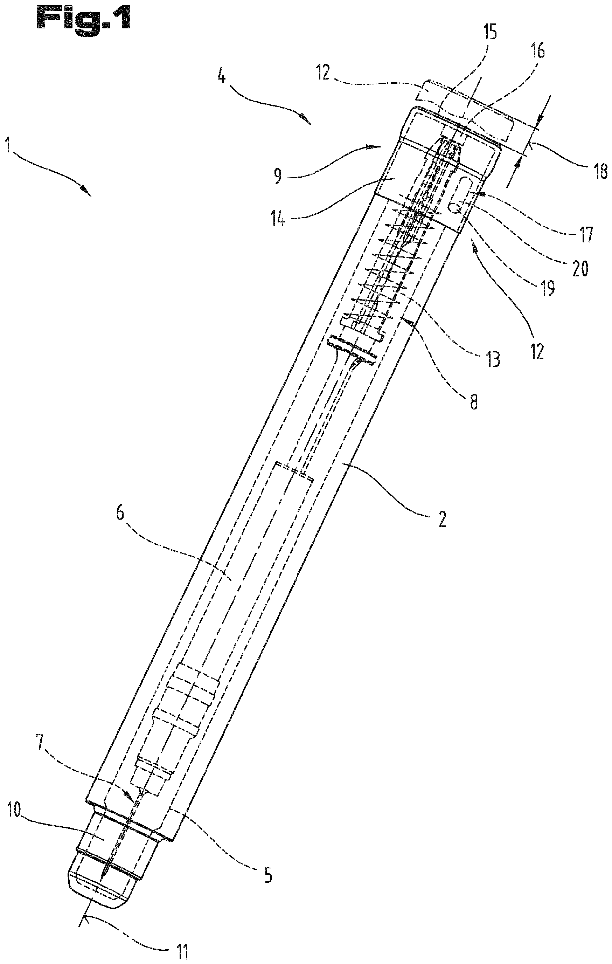

In den Fig. 1 bis 6 ist jeweils eine Injektionsvorrichtung 1 zur Abgabe eines Medikaments oder einer Injektionsflüssigkeit gezeigt, wobei die Injektionsvorrichtung 1 zur automatisierten Abgabe ausgebildet ist. Dabei kann als übliche Fachbezeichnung der Begriff Autoinjektor gewählt werden. Die einzelnen Ausbildungen in den Fig. 1 bis 6 weisen zueinander nur geringfügige Unterschiede auf, wobei jedoch jede der Ausbildungen eine eigene, für sich unabhängige Ausführungsform darstellen kann, aber auch einzelne Detailausbildungen unterschiedlicher Ausführungsformen miteinander kombiniert werden können.

So sind die unterschiedlichsten automatisierten Injektionsvorrichtungen 1 bekannt, wobei diese stets von einer Aufbewahrungsstellung in eine Injektionsstellung selbsttätig verstellbar ausgebildet sind. Dabei erfolgt sowohl der Einstichvorgang als auch der Abgabevorgang der abzugebenden Injektionsflüssigkeit oder des Medikaments selbsttätig und erst nach Freigabe und gezielter Auslösung durch den Benutzer.

Um eine Infektion oder Übertragung von Krankheiten im Zuge des Einstichvorgangs und dem damit verbundenen Abgabevorgang zu vermeiden, sind zumindest jene beim Einstichvorgang in den Körper eindringenden Bauteile während der Aufbewahrungsstellung in einem sterilen Zustand zu halten.

Allgemein umfasst die Injektionsvorrichtung 1 eine Vielzahl von einzelnen Bauteilen sowie aus diesen gebildete Baugruppen. So kann die Injektionsvorrichtung 1 ein zumeist hülsenförmig ausgebildetes Basisgehäuse 2 mit einem dem Patienten zuwendbaren distalen Ende 3 und einem davon abgewendeten proximalen Ende 4 umfassen. Weiters kann als Bezeichnung für die Ortsangabe vorne und hinten verwendet werden, wobei "vorne" dem distalen Ende 3 und "hinten" dem proximalen Ende 4 zugeordnet ist. Je nach Ausbildung der Injektionsvorrichtung 1 kann das Basisgehäuse 2 auch noch als Auslösehülse oder Aktivierungshülse bezeichnet werden. Dies ist bei jener Ausführung der Fall, bei welcher durch die Relativverlagerung des Basisgehäuses 2 in Bezug auf die Einstichstelle in bekannter Weise die Auslösung der Injektionsvorrichtung 1 und damit verbunden die Abgabe des fleißfähigen Medikaments bzw. der Injektionsflüssigkeit erfolgt.

Wie nun aus der schematisch vereinfachten Darstellung der Fig. 1 zu ersehen ist, kann innerhalb des Basisgehäuses 2 ein eigenes Traggehäuse 5 zumindest teilweise aufgenommen bzw. angeordnet sein. Weiters kann die Injektionsvorrichtung 1 auch noch ein Speichergefäß, wie eine Karpule 6, eine Nadelanordnung 7, zumindest eine Antriebsbaugruppe 8 sowie eine Sicherungsbaugruppe 9 umfassen. Die Nadelanordnung 7 umfasst eine Kanüle, welche an einem Ende zum Einstich in den Körper vorgesehen ist und am anderen Ende mit der Injektionsflüssigkeit in der Karpule 6 bereits vor dem Injektionsvorgang in Strömungsverbindung steht oder erst im Zuge des Injektionsvorgangs mit dem Innenraum der Karpule 6 in Strömungsverbindung gebracht wird.

Das Aufnahmegefäß für die Injektionsflüssigkeit ist hier durch die Karpule 6 gebildet, in der das Medikament, die Injektionsflüssigkeit bzw. der abzugebende und zu injizierende Wirkstoff bereits während der Aufbewahrungsstellung darin bevorratet und abgabefertig vorliegt. So ist es möglich, dass die Karpule 6, die Nadelanordnung 7, die zumindest eine Antriebsbaugruppe 8 sowie Teile der Sicherungsbaugruppe 9 zumindest bereichsweise im Basisgehäuse 2 aufgenommen oder daran angeordnet sind. Ist ein eigenes Traggehäuse 5 vorgesehen, können die zuvor aufgezählten Bauteile auch in diesem aufgenommen oder angeordnet sein. Das Nadelschutzelement 10 kann in radialer Richtung gesehen zwischen dem Traggehäuse 5 und dem Basisgehäuse 2 angeordnet sein.

Die Antriebsbaugruppe 8 steht mit der Karpule 6 in Wirkverbindung, um zumindest die Abgabe des Medikaments bzw. der Injektionsflüssigkeit nach erfolgtem Einstich durchzuführen. Um nach erfolgter Abgabe und bereits vom Körper entfernter Injektionsvorrichtung 1 ungewollte Stichverletzungen zu vermeiden, kann diese auch noch ein Nadelschutzelement 10 umfassen, welches zumindest den gebrauchten Kanülenabschnitt sicher abdeckt. Dazu kann eine weitere auslösbare Antriebseinheit vorgesehen sein, welche der besseren Übersichtlichkeit nicht dargestellt worden ist. Das Nadelschutzelement 10 mitsamt deren weiteren Antriebseinheit kann, muss aber nicht vorgesehen sein.

Zwischen dem distalen Ende 3 und dem proximalen Ende 4 des Basisgehäuses 2 erstreckt sich eine Längsachse 11. Die Längsachse 11 kann aber auch für die gesamte Injektionsvorrichtung 1 als gemeinsame Achse angesehen werden.

Die Nadelanordnung 7 ist in der Aufbewahrungsstellung zumeist der Karpule 6 vorgeordnet angeordnet und befindet sich im vorderen Bereich und somit im Bereich des distalen Endes 3. Die Antriebsbaugruppe 8 umfasst zumindest ein Antriebsmittel zur automatischen Abgabe des abzugebenden Medikaments bzw. der Injektionsflüssigkeit, welches Antriebsmittel zumeist durch eine Druckfeder gebildet ist. Das Antriebsmittel ist durch die Sicherungsbaugruppe 9 so lange gegen eine Freigabe gesichert ist, bis dass durch einen Benutzer bewusst eine Aktivierung der Injektionsvorrichtung 1 erfolgt, bei der die Antriebsbaugruppe 8 freigegeben und damit die Injektionsvorrichtung 1 ausgelöst wird. Durch die Sicherungsbaugruppe 9 ist somit die erste Antriebsbaugruppe bis zu deren Aktivierung für den Injektionsvorgang bezüglich des Basisgehäuses 2 in ihrer Position festgelegt.

Es ist möglich, dass das Basisgehäuse 2 in einer geteilten Ausführung ausgebildet sein kann. So kann im Bereich des distalen Endes 3 der Injektionsvorrichtung 1 die Karpule 6, die Nadelanordnung 7 sowie gegebenenfalls auch noch das Nadelschutzelement 10 mitsamt deren weiteren Antriebseinheit angeordnet oder aufgenommen sein. Die Antriebsbaugruppe 8 und die Sicherungsbaugruppe 9 können in jenem Teil des Basisgehäuses 2 aufgenommen bzw. angeordnet sein, welcher das proximale Ende 4 aufweist bzw. ausbildet. Damit kann jener Teil der Injektionsvorrichtung 1, in welchem das Medikament bzw. der Injektionsflüssigkeit aufgenommen ist, nach Ablauf eines Ablaufdatums gegen einen neuen Teil mit einem frischen Medikament bzw. einer frischen Injektionsflüssigkeit ersetzt werden. Jener Teil mit der Antriebsbaugruppe 8 und der Sicherungsbaugruppe 9 kann weiter verwendet werden.

Den wesentlichen Teil der hier beschriebenen Injektionsvorrichtung 1 bildet die Sicherungsbaugruppe 9 für das Zusammenwirken mit der Antriebsbaugruppe 8.

Die Sicherungsbaugruppe 9 legt die Position der zumindest einen Antriebsbaugruppe 8 bis zu deren Aktivierung für den Injektionsvorgang bezüglich des Basisgehäuses 2 fest. Im vorliegenden Ausführungsbeispiel umfasst die Sicherungsbaugruppe 9 eine Sicherungskappe 12 sowie zumindest einen Haltearm 13. Der Haltearm 13 stellt in bekannter Weise das Verbindungsglied zur Antriebsbaugruppe 8 dar. Der oder die Haltearme 13 ist oder sind in einer verrasteten Position im Bereich des proximalen Endes 4 an einer Haltescheibe eingerastet gehalten und durch einen Sicherungsstift 16 bis zu deren Auslösung daran arretiert gehalten. Der Sicherungsstift 16 ist in bekannter Weise innerhalb des zumindest einen Haltearms 13, bevorzugt im axialen Zentrum der Sicherungsbaugruppe 9, angeordnet und hindert den oder die Haltearme 13 an einer radialen Verstellung nach innen in Richtung auf die Längsachse 11. Erst durch die in proximaler Richtung erfolgte Rückstellung der Sicherungskappe 12 mitsamt dem Sicherungsstift 16 das Entfernen desselben aus dem Kontaktbereich mit dem zumindest einen Haltearm 13 kann der zumindest eine Haltearm 13 aus seiner verrasteten Stellung verstellt werden, um so anschließend die Antriebsbaugruppe 8 aktivieren zu können. Bei dem vorliegenden Ausführungsbeispiel ist das Basisgehäuse 2 im Bereich seines proximalen Endes dazu ausgebildet, bei einer relativen Verlagerung den zumindest einen Haltearm 13 aus seiner verrasteten Stellung in radialer Richtung in den durch die Entfernung des Sicherungsstifts 16 geschaffenen Freiraum zu verlagern und damit die Aktivierung der Antriebsbaugruppe 8 zu bewirken.

Die Sicherungskappe 12 weist ihrerseits einen Kappenmantel 14 sowie eine Kappenwand 15 auf. Der Kappenmantel 14 ist bevorzugt über den Umfang durchlaufend ausgebildet, wobei die Kappenwand 15 auch als Stirnwand oder Deckwand bezeichnet werden kann. Innerhalb des Kappenmantels 14 ist an der Kappenwand 15, insbesondere in dessen Zentrum, der sich in Richtung der Längsachse 11 erstreckende und bevorzugt auch im Zentrum des Kappenmantels 14 angeordnete Sicherungsstift 16 angeordnet. Bevorzugt ist der Sicherungsstift 16 als bolzenförmiger Bauteil ausgebildet, wobei seine Achse oder Mittelachse in der Längsachse 11 liegend ausgerichtet ist. Die Kappenwand 15 ist zur Befestigung des Sicherungsstifts 16 zumindest im Bereich ihres Zentrumabschnitts durchgängig und vollständig ausgebildet.

Die Sicherungskappe 12 ist in der Aufbewahrungsstellung der Injektionsvorrichtung 1 am Basisgehäuse 2 in einer Sicherungsstellung für die zumindest eine Antriebsbaugruppe 8 gehalten bzw. angeordnet. Dabei hält der Sicherungsstift 16 in der Sicherungsstellung zumindest den einen Haltearm 13 der Sicherungsbaugruppe 9 in radialer Richtung bezüglich der Längsachse 11 in einer verrasteten Position ortsfest positioniert. Dies ist hinlänglich bekannt, wobei erst nach dem Entfernen des Sicherungsstifts 16 aus dem Bereich des oder der Haltearme 13 deren verrastete Position durch einen entsprechenden Aktivierungsvorgang freigegeben werden und damit die Antriebsbaugruppe 8 in weiterer Folge aktiviert werden kann.

Die Sicherungskappe 12 kann in Axialrichtung relativ bezüglich des Basisgehäuses 2 ausgehend von ihrer Sicherungsstellung in eine Freigabestellung verstellt werden, bei welcher der Sicherungsstift 16 außer Eingriff mit dem oder den Haltearmen 13 kommt.

Bei den bislang bekannten Sicherungsvorrichtungen bei derartigen Injektionsvorrichtungen 1 wurde zur Freigabe der Antriebsbaugruppe 8 die Sicherungskappe vollständig abgezogen. Damit ist im Bereich des proximalen Endes 4 jene den Sicherungsstift aufnehmende Öffnung sichtbar geworden, was zu Fehlbedienungen führen konnte. Da nun sowohl im Bereich des distalen Endes 3 als auch im Bereich des proximalen Endes 4 jeweils eine Öffnung zu ersehen war, war es für manche Anwender nicht eindeutig, welches der beiden Enden für den Injektionsvorgang der jeweiligen Körperstelle zuzuwenden war.

So ist hier vorgesehen, dass zwischen der Sicherungskappe 12 und dem Basisgehäuse 2 eine eigene Führungsanordnung 17 vorgesehen oder ausgebildet ist. Damit kann ein in Axialrichtung begrenzter Verstellweg 18 der Sicherungskappe 12 zwischen deren Sicherungsstellung und deren Freigabestellung relativ bezüglich des Basisgehäuses 2 festgelegt werden. Die Verstellrichtung Sicherungskappe 12 erfolgt ausgehend von einer dem distalen Ende 3 näherliegenden Position in eine dazu weiter entfernte Position. Die damit verbundene Verstellrichtung ist damit so gewählt, dass diese auf die vom distalen Ende 3 abgewendete Seite oder Richtung erfolgt. Die hier gewählte und auch zumeist übliche Verstellrichtung der Sicherungskappe 12 kann auch als in proximaler Richtung erfolgend bezeichnet werden. Durch diesen begrenzten Verstellweg 18 ist die Sicherungskappe 12 auch in deren Freigabestellung noch am Basisgehäuse 2 angeordnet oder gehalten, jedoch der vom Sicherungsstift 16 zumindest eine Haltearm 13 für die Auslösung der Antriebsbaugruppe 8 freigegeben. Die Freigabestellung der Sicherungskappe 12 ist in strich-punktierten Linien angedeutet. Weiters ist vereinfacht ein erstes Führungselement 19 und zumindest ein damit zusammenwirkendes zweites Führungselement 20 angedeutet. Die möglichen Ausbildungen derselben sind nachfolgend näher beschrieben.

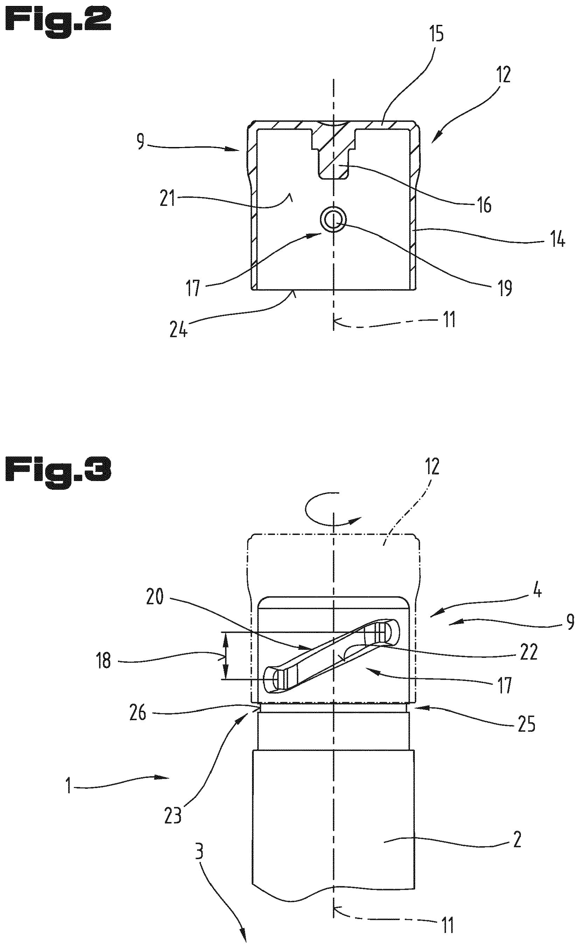

Wie nun besser aus den Fig. 2 und 3 zu ersehen ist, kann die Führungsanordnung 17 zumindest ein erstes Führungselement 19 und zumindest ein damit zusammenwirkendes zweites Führungselement 20 umfassen. Diese beiden Führungselemente 19, 20 dienen dazu, den in Axialrichtung begrenzten Verstellweg 18 zwischen der Sicherungskappe 12 und dem Basisgehäuse 2 festzulegen und zu begrenzen. Je nach Ausbildung der Führungselemente 19, 20 können diese aber auch als Anschläge oder Fortsätze bezeichnet werden, welche die jeweilige Stellung, nämlich die Freigabestellung und/oder die Sicherungsstellung in ihrer Position relativ bezüglich des Basisgehäuses 2 festlegen. Im vorliegenden Ausführungsbeispiel ist das zumindest eine erste Führungselement 19 durch einen Fortsatz gebildet, welcher an einer Innenfläche 21 des Kappenmantels 14 angeordnet oder ausgebildet ist. Dabei ragt der zumindest eine, das erste Führungselement 19 bildende Fortsatz in Richtung auf die Längsachse 11 in radialer Richtung vor.

Das zumindest eine zweite Führungselement 20 kann durch eine am oder im Basisgehäuse 2 ausgebildete Führungsbahn 22 gebildet sein. So ist es möglich die Führungsbahn 22 an der Außenseite des Basisgehäuses 2 vertieft in dieser auszubilden oder anzuordnen. Weiters kann die Führungsbahn 22 am Basisgehäuse im Bereich des proximalen Endes 4 angeordnet oder ausgebildet sein.

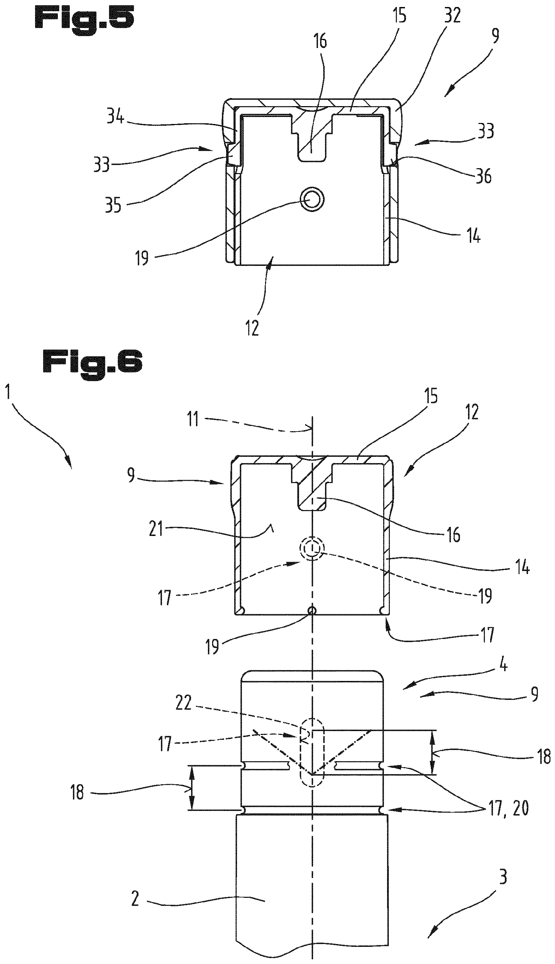

Der Längsverlauf der Führungsbahn 22 kann dabei unterschiedlichst gewählt bzw. ausgebildet sein. Als einfachste und kürzeste Verstellung kann sich beispielsweise die Führungsbahn 22 ausschließlich in Axialrichtung erstrecken, wodurch keinerlei relative Drehbewegung der Sicherungskappe 12 während deren Verstellbewegung am Basisgehäuse stattfinden kann. Dies ist in der nachfolgenden Fig. 6 noch näher gezeigt und beschrieben.

Es wäre aber auch möglich, dass die Führungsbahn 22 in deren Längsverlauf bezüglich einer in senkrechter Richtung zur Längsachse 11 ausgerichteten Ebene geneigt verlaufend ausgebildet ist. Damit kann durch eine Dreh- bzw. Schwenkbewegung der Sicherungskappe 12 um die Längsachse 11 des Basisgehäuses 2 ebenfalls der axiale Verstellweg 18 zurückgelegt werden. Die Steigung der Führungsbahn 22 bezüglich der in senkrechter Richtung zur Längsachse 11 ausgerichteten Ebene kann beispielsweise ausgehend von der Sicherungsstellung hin zur Freigabestellung gleichmäßig geneigt verlaufen.

Es wäre aber auch möglich, wie dies in der Fig. 3 zu ersehen ist, dass die Führungsbahn 22 in deren Längsverlauf bezüglich der in senkrechter Richtung zur Längsachse 11 ausgerichteten Ebene eine unterschiedliche Steigung mit zueinander unterschiedlichen Steigungswinkeln aufweist. So könnte der Steigungswinkel der Führungsbahn 22 im Bereich der Sicherungsstellung und/oder der Freigabestellung flacher ausgebildet sein als in jenem sich zwischen der Sicherungsstellung und der Freigabestellung erstreckenden Zwischenabschnitt. So könnte beispielsweise im Bereich der Sicherungsstellung und/oder der Freigabestellung der Steigungswinkel der Führungsbahn 22 parallel bezüglich jener in senkrechter Richtung zur Längsachse 11 ausgerichteten Ebene verlaufen.

Weiters ist es noch möglich, dass die Sicherungsbaugruppe 9 weiters eine Anzeigeeinheit 23 umfasst. Mittels der Anzeigeeinheit 23 wird es möglich, zumindest eine der beiden Stellungen der Sicherungskappe 12 relativ bezüglich des Basisgehäuses 2 dem Benutzer anzuzeigen. So kann beispielsweise die Anzeigeeinheit 23 zumindest ein erstes Anzeigemittel 24 umfassen, welches im Bereich der Sicherungskappe 12 angeordnet oder ausgebildet ist. Im einfachsten Fall könnte das erste Anzeigemittel 24 beispielsweise durch einen von der Kappenwand 15 distanziert angeordneten Kappenrand des Kappenmantels 14 gebildet sein. Ein zweites Anzeigemittel 25 der Anzeigeeinheit 23 kann bevorzugt im Bereich des Basisgehäuses 2 angeordnet oder ausgebildet sein.

Wird beispielsweise der Kappenrand der Kappenwand 15 als erstes Anzeigemittel 24 gewählt, kann beispielsweise das zweite Anzeigemittel 25 durch eine über den Umfang des Basisgehäuses 2 verteilt oder durchlaufend ausgebildete Markierung gebildet sein. Diese Markierung kann beispielsweise durch einen färbigen Ring, eine Nut oder dergleichen gebildet sein.

Es wäre aber auch noch möglich, dass das zumindest eine erste Anzeigemittel 24 durch einen Durchbruch oder ein Fenster im Kappenmantel 14 und/oder der Kappenwand 15 gebildet ist, wie dies nachfolgend noch gezeigt und beschrieben wird.

Weiters ist hier noch zu ersehen, dass am Basisgehäuse 2 das zweite Anzeigemittel 25 durch einen Markierungsring, insbesondere eine nutförmige Vertiefung 26 gebildet ist. Somit kommt das zweite Anzeigemittel 25 nach der axialen Verstellung der Sicherungskappe 12 um den sich in Axialrichtung erstreckenden Verstellweg 18 unterhalb des Kappenrandes des Kappenmantels 14 zum Vorschein, wie dies in der Fig. 3 durch die in strichpunktierten Linie angedeutete Sicherungskappe 12 veranschaulicht ist. Der axiale Verstellweg 18 wird hier durch eine Dreh- bzw. Schwenkbewegung um einen Verschwenkwinkel mittels der zusammenwirkenden ersten und zweiten Führungselemente 19, 20 zurück gelegt.

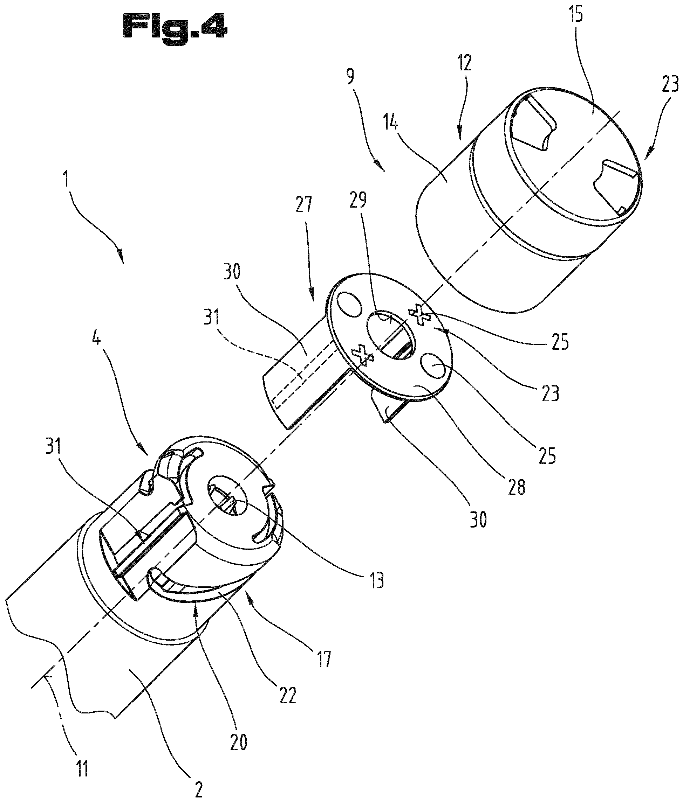

In der Fig. 4 ist eine weitere und gegebenenfalls für sich eigenständige Ausführungsform der Injektionsvorrichtung 1, insbesondere deren Sicherungsbaugruppe 9 gezeigt, wobei wiederum für gleiche Teile gleiche Bezugszeichen bzw. Bauteilbezeichnungen wie in den vorangegangenen Fig. 1 bis 3 verwendet werden. Um unnötige Wiederholungen zu vermeiden, wird auf die detaillierte Beschreibung in den vorangegangenen Fig. 1 bis 3 hingewiesen bzw. Bezug genommen.

Die hier gezeigte Ausführungsform der Sicherungsbaugruppe 9 ist dabei ähnlich ausgebildet wie bereits zuvor detailliert in den Fig. 1 bis 3 beschrieben. Deshalb wird hier lediglich auf die Ausbildung der Sicherungsbaugruppe 9 im Bereich des proximalen Endes 4 des Basisgehäuses 2 näher eingegangen.

Auch hier ist wiederum die Sicherungskappe 12 mit dem Kappenmantel 14, mit der Kappenwand 15 sowie mit dem im Inneren, jedoch nicht näher dargestellten Sicherungsstift 16 , vorhanden. Die Ausbildung der Führungsanordnung 17 ist auch gleichartig wie in den Fig. 2 und 3 gewählt. Das oder die ersten Führungselemente 19 sind wiederum an der Innenfläche 21 des Kappenmantels 14 angeordnet, jedoch wurden diese nicht mehr näher dargestellt. Das oder die zweiten Führungselemente 20 sind durch jeweils eigene Führungsbahnen 22 gebildet, wobei deren Längsverlauf wiederum den axialen Verstellweg 18 festlegt, wie dies bereits zuvor beschrieben worden ist.

Die Anzeigeeinheit 23 ist hier im Gegensatz zu der zuvor beschriebenen Ausführungsform dazu unterschiedlich ausgebildet. So umfasst die Anzeigeeinheit 23 ein zusätzliches Anzeigeträgerelement 27, welches im Einbauzustand innerhalb des Kappenmantels 14 sowie unmittelbar benachbart zur Kappenwand 15 und damit innerhalb der Sicherungskappe 12 angeordnet bzw. aufgenommen ist. Weiters ist das Anzeigeträgerelement 27 am Basisgehäuse 2 in Axialrichtung verstellbar geführt, um so eine Verlagerung bzw. Verstellung in Axialrichtung, jedoch eine Drehung bzw. Verschwenkung um die Längsachse 11 zu verhindern bzw. nicht zuzulassen.

Die Sicherungskappe 12 ist ihrerseits relativ bezüglich des Anzeigeträgerelements 27 um die Längsachse 11 verschwenkbar oder verdrehbar. Das Ausmaß des Verschwenk- bzw. Verdrehwinkels der Sicherungskappe 12 relativ bezüglich des Basisgehäuses 2 ist dabei durch die Führungsanordnung 17, insbesondere der Umfangserstreckung der Führungsbahn 22 definiert bzw. festgelegt. Weiters ist das Anzeigeträgerelement 27 an der Sicherungskappe 12 relativ bezüglich dieser in Richtung der Längsachse 11 positioniert daran gehalten. Dies kann durch nicht näher bezeichnete Haltenasen oder eine nutförmige Vertiefung, welche in der Innenfläche 21 des Kappenmantels 14 angeordnet ist, erfolgen. Somit kann das Anzeigeträgerelement 27 gemeinsam mit der Sicherungskappe 12 die Verstellung entlang des axialen Verstellwegs 18 gemeinsam mit durchführen, jedoch ist eine relative Verschwenkung bzw. Verdrehung des Anzeigeträgerelements 27 um die Längsachse 11 des Basisgehäuses 2 verhindert.

So umfasst bei diesem Ausführungsbeispiel das Anzeigeträgerelement 27 einen scheibenförmig ausgebildeten Grundkörper 28 mit einer in dessen Zentrum angeordneten Durchbrechung 29. Die Durchbrechung 29 dient zur Aufnahme bzw. zum Hindurchführen des in der Sicherungskappe 12 angeordneten Sicherungsstifts 16. Des Weiteren umfasst das Anzeigeträgerelement 27 zumindest einen sich in Axialrichtung davon erstreckenden oder davon abstehenden Führungssteg 30. Dabei kann der zumindest eine Führungssteg 30 im äußeren Umfangsbereich des scheibenförmig ausgebildeten Grundkörpers 28 angeordnet sein. Bevorzugt sind zwei derartiger Führungsstege 30 vorgesehen, um eine einwandfreie Axialführung des Anzeigeträgerelements 27 am Basisgehäuse 2 sicherzustellen. Die beiden Führungsstege 30 sind in einer zueinander diametral einander gegenüberliegenden Anordnung am scheibenförmig ausgebildeten Grundkörper 28 angeordnet. Zur Aufnahme des oder der Führungsstege 30 sind dazu gegengleich am Basisgehäuse 2 ausgebildete Abflachungen vorgesehen, um so innerhalb der Sicherungskappe 12 auch die beiden Führungsstege 30 aufnehmen zu können.

Zusätzlich kann zwischen dem zumindest einen Führungssteg 30 und dem Basisgehäuse 2 eine Längsführungsanordnung 31 angeordnet oder ausgebildet sein. Die Längsführungsanordnung 31 kann zusammenwirkende bzw. miteinander in Eingriff stehende Längsführungselemente aufweisen, welche nicht näher bezeichnet sind. Diese können beispielsweise durch eine nutförmige Vertiefung und einen in diese eingreifenden Steg oder dergleichen gebildet sein.

Die Anzeigeeinheit ist bei diesem hier gezeigten Ausführungsbeispiel durch zumindest ein erstes Anzeigemittel 24 gebildet, welches als Durchbruch in der Kappenwand 15 der Sicherungskappe 12 ausgebildet ist. Im vorliegenden Ausführungsbeispiel sind zwei Durchbrüche vorgesehen, wobei diese beiden Durchbrüche diametral zueinander in der Kappenwand 15 angeordnet sind.

Die Anzeigeeinheit 23 umfasst hier weiters das zumindest zweite Anzeigemittel 25, welches am Anzeigeträgerelement 27, insbesondere am scheibenförmig ausgebildeten Grundkörper 28 angeordnet oder ausgebildet ist. Dabei ist das oder sind die zweiten Anzeigemittel 25 auf jener Seite des Anzeigeträgerelements 27 angeordnet, welche der Kappenwand 15 sowie dem oder den in dieser angeordneten Durchbrüche zugewendet ist.

Das zumindest eine zweite Anzeigemittel 25 kann aus der Gruppe von Symbolen, Farbkennung, Markierungsring, Bild, nutförmige Vertiefung, Blindenschrift, Brailleschrift gebildet und/oder ausgewählt sein.

Bei den Symbolen oder Bildern könnte beispielsweise ein offenes oder geschlossenes Schloss, unterschiedliche Farbgebungen oder dergleichen gewählt sein. Damit soll dem Benutzer eindeutig klar signalisiert werden, dass bei sich in der Sicherungsstellung befindlicher Sicherungskappe die Injektionsvorrichtung 1 noch in deren Aufbewahrungsstellung befindet und somit eine Freigabe bzw. Betätigung der Antriebsbaugruppe 8 nicht möglich ist. Erst nach durchgeführter Verstellung der Sicherungskappe 12 in Axialrichtung wird in der entsprechenden Freigabestellung dem Benutzer durch das Sichtbarwerden bzw. Erscheinen des zweiten Anzeigemittels 25 eindeutig klar signalisiert, dass die Injektionsvorrichtung 1 für die Aktivierung der Antriebsbaugruppe 8 und der damit verbundenen Abgabe des Medikaments bzw. der Injektionsflüssigkeit bereit ist.

In der Fig. 5 ist eine weitere und gegebenenfalls für sich eigenständige Ausführungsform der Injektionsvorrichtung 1, insbesondere deren Sicherungsbaugruppe 9 gezeigt, wobei wiederum für gleiche Teile gleiche Bezugszeichen bzw. Bauteilbezeichnungen wie in den vorangegangenen Fig. 1 bis 4 verwendet werden. Um unnötige Wiederholungen zu vermeiden, wird auf die detaillierte Beschreibung in den vorangegangenen Fig. 1 bis 4 hingewiesen bzw. Bezug genommen.

Da bei all den hier gezeigten und beschriebenen Ausführungsformen grundsätzlich die Sicherungskappe 12 auch in der Freigabestellung weiterhin am Basisgehäuse 2 angeordnet bzw. gehalten ist, wird für den Benutzer die Möglichkeit geschaffen, dass trotzdem ein kappenähnlicher Teil für die Freigabe der Injektionsvorrichtung 1 vom Basisgehäuse 2 abgenommen werden kann. Dazu ist vorgesehen, dass die Sicherungsbaugruppe 9 weiters eine Betätigungskappe 32 umfasst, welche lösbar an der Sicherungskappe 12 gehalten ist. Die Betätigungskappe 32 übergreift die Sicherungskappe 12 im Bereich ihres Kappenmantels 14 und kann diese insbesondere vollständig in sich aufnehmen. Weiters können zwischen der Betätigungskappe 32 und der Sicherungskappe 12, insbesondere im Bereich des Kappenmantels 14 derselben, miteinander in Eingriff stehende Rastmittel 33 vorgesehen sein.

So wäre es möglich, an der Sicherungskappe 12 als Rastmittel 33 zumindest einen an einem Federarm 34 angeordneten Rastfortsatz 35 vorzusehen. Als weiteres Rastmittel 33 kann an der Betätigungskappe 32, insbesondere dessen Kappenmantel, zumindest eine den Rastfortsatz 35 aufnehmende Rastausnehmung 36 angeordnet bzw. vorgesehen sein.

Da der zumindest eine Rastfortsatz 35 an einem Federarm 34 angeordnet ist, wird es für einen Benutzer möglich, nach Überwinden der Rastkraft die Betätigungskappe 32 außer Eingriff mit dem oder den Rastfortsätzen 35 der Sicherungskappe 12 zu bringen und damit die Betätigungskappe 32 von der Sicherungskappe 12 entfernen zu können.

In der Fig. 6 sind zwei weitere und gegebenenfalls für sich eigenständige Ausführungsformen der Injektionsvorrichtung 1, insbesondere deren Sicherungsbaugruppen 9 gezeigt, wobei wiederum für gleiche Teile gleiche Bezugszeichen bzw. Bauteilbezeichnungen wie in den vorangegangenen Fig. 1 bis 5 verwendet werden. Um unnötige Wiederholungen zu vermeiden, wird auf die detaillierte Beschreibung in den vorangegangenen Fig. 1 bis 5 hingewiesen bzw. Bezug genommen.

Wie bereits zuvor kurz beschrieben, sind hier noch weitere mögliche Anordnungen und Ausbildungen von Führungsanordnungen 17 zur Festlegung bzw. Definition des axialen Verstellwegs 18 gezeigt.

In strichlierten Linien ist jene Ausführungsform gezeigt, bei welcher nur eine reine geradlinige, axiale Verstellung zwischen der Sicherungskappe 12 und dem Basisgehäuse 2 möglich ist. Das erste Führungselement 19 ist wiederum an der Innenfläche 21 des Kappenmantels 14 angeordnet. Der Längsverlauf der das zweite Führungselement 20 bildenden Führungsbahn 22 ist dabei als nutförmige Vertiefung, jedoch mit einer reinen Axialerstreckung, am Basisgehäuse 2 ausgebildet.

Bei jener in vollen Linien gezeigten Ausführungsform sind im Bereich des offenen Endes des Kappenmantels 14, insbesondere im Bereich von dessen Kappenrand, das oder die ersten Führungselemente 19 als einzelne Vorsprünge ausgebildet. Es wäre aber auch möglich, das erste Führungselement 19 durch einen umlaufenden, in Richtung auf die Längsachse 11 vorragenden Bund oder flanschförmigen Ansatz auszubilden.

Das zweite Führungselement 20 ist bevorzugt in zweifacher Ausführung am Basisgehäuse 2 als nutförmige Vertiefung ausgebildet. Der Axialabstand entspricht dabei dem vorgesehenen axialen Verstellweg 18. Damit wird sowohl in der Sicherungsstellung der Sicherungskappe 12 als auch in deren Freigabestellung eine positionierte Halterung der Sicherungskappe 12 im Bereich des proximalen Endes 4 des Basisgehäuses 2 erzielt. Es wäre aber auch möglich, nur eine der beiden dargestellten nutförmigen Vertiefungen als zweites Führungselement 20 vorzusehen.

Auch bei diesem Ausführungsbeispiel kann wiederum die Anzeigeeinheit 23 mit zumindest einem ersten Anzeigemittel 24 und zumindest einem zweiten Anzeigemittel 25 vorgesehen sein. Die Ausbildung kann analog erfolgen, wie dies bereits zuvor beschrieben worden ist. Da hier bevorzugt keine umfangsmäßige, relative Verstellung zwischen dem Basisgehäuse 2 und der Sicherungskappe 12 vorgesehen ist, kann jene Ausführungsform der Anzeigeeinheit 23 gewählt werden, wie diese insbesondere in der Fig. 2 beschrieben worden ist.

Wird als zweites Führungselement 20 eine Führungsbahn 22 gewählt, könnte diese auch so ausgebildet sein, dass ausgehend von der Sicherungsstellung eine Schwenk- bzw. Drehbewegung sowohl im Uhrzeigersinn als auch entgegen diesem ermöglicht wird. Ist einer der beiden möglichen Drehsinne gewählt und die Sicherungskappe 12 in die Freigabestellung verstellt worden, ist der in Axialrichtung begrenzte Verstellweg 18 zurückgelegt.

Zusätzlich oder unabhängig davon könnte bei all den zuvor beschriebenen Ausführungen auch noch vorgesehen sein, dass bei Erreichen der Freigabestellung eine zusätzliche Verrastung zwischen den Führungselementen 19, 20 erzielt wird. Dazu könnte in der Führungsbahn 22 ein nicht näher gezeigter Vorsprung oder Ansatz und/oder eine Vertiefung zur Aufnahme des ersten Führungselements 19 vorgesehen sein.

Eine weitere Anwendungsvariante soll auch noch möglich sein, bei welcher die Sicherungskappe 12 noch vor der Aktivierung der Antriebsbaugruppe 8 von deren Freigabestellung wieder zurück in die Sicherungsstellung verstellt werden kann. Dies ermöglicht einem Benutzer, dass bei noch nicht ausgelöster Antriebsbaugruppe 8 die Aufbewahrungsstellung der Injektionsvorrichtung 1 wieder hergestellt werden kann.

Der äußere Querschnitt des Kappenmantels 14 kann auch mit Abflachungen und/oder Fortsätzen versehen sein und/oder auch einen mehreckigen Außenquerschnitt aufweisen, um damit ein Wegrollen der Injektionsvorrichtung 1 verhindern zu können.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

### Bezugszeichenaufstellung

| | | | |
|---|---|---|---|
| 1 | Injektionsvorrichtung | 31 | Längsführungsanordnung |
| 2 | Basisgehäuse | 32 | Betätigungskappe |
| 3 | Ende | 33 | Rastmittel |
| 4 | Ende | 34 | Federarm |
| 5 | Traggehäuse | 35 | Rastfortsatz |
| 6 | Karpule | 36 | Rastausnehmung |
| 7 | Nadelanordnung | | |
| 8 | Antriebsbaugruppe | | |
| 9 | Sicherungsbaugruppe | | |
| 10 | Nadelschutzelement | | |
| 11 | Längsachse | | |
| 12 | Sicherungskappe | | |
| 13 | Haltearm | | |
| 14 | Kappenmantel | | |
| 15 | Kappenwand | | |
| 16 | Sicherungsstift | | |
| 17 | Führungsanordnung | | |
| 18 | Verstellweg | | |
| 19 | erstes Führungselement | | |
| 20 | zweites Führungselement | | |
| 21 | Innenfläche | | |
| 22 | Führungsbahn | | |
| 23 | Anzeigeeinheit | | |
| 24 | erstes Anzeigemittel | | |
| 25 | zweites Anzeigemittel | | |
| 26 | Vertiefung | | |
| 27 | Anzeigeträgerelement | | |
| 28 | Grundkörper | | |
| 29 | Durchbrechung | | |
| 30 | Führungssteg | | |

## Patentansprüche

1. Injektionsvorrichtung (1), welche als Autoinjektor zur Abgabe einer Injektionsflüssigkeit ausgebildet ist, wobei die Injektionsvorrichtung (1) von einer Aufbewahrungsstellung in eine Injektionsstellung verstellbar ausgebildet ist, mit
- einem hülsenförmig ausgebildeten Basisgehäuse (2), welches Basisgehäuse (2) ein einem Patienten zuwendbares distales Ende (3) und ein davon abgewendetes proximales Ende (4) aufweist, zwischen welchen Enden (3, 4) sich eine Längsachse (11) erstreckt,
- einer Karpule (6) mit der darin enthaltenen und beim Injektionsvorgang abzugebenden Injektionsflüssigkeit,
- einer Nadelanordnung (7), die der Karpule (6) in der Aufbewahrungsstellung der Injektionsvorrichtung (1) im Abschnitt des distalen Endes (3) vorgeordnet ist,
- zumindest einer Antriebsbaugruppe (8), welche Antriebsbaugruppe (8) ein Antriebsmittel zur automatischen Abgabe der abzugebenden Injektionsflüssigkeit beim Injektionsvorgang umfasst,
- einer Sicherungsbaugruppe (9), welche Sicherungsbaugruppe (9) die zumindest eine Antriebsbaugruppe (8) bis zu deren Aktivierung für den Injektionsvorgang bezüglich des Basisgehäuses (2) in ihrer Position festlegt,
- wobei die Sicherungsbaugruppe (9) eine Sicherungskappe (12) sowie zumindest einen Haltearm (13) umfasst, wobei die Sicherungskappe (12) einen Kappenmantel (14), eine Kappenwand (15) sowie einen innerhalb des Kappenmantels (14) sowie in einem Zentrum an der Kappenwand (15) angeordneten und sich in Richtung der Längsachse (11) erstreckenden Sicherungsstift (16) umfasst,
- und wobei die Sicherungskappe (12) in der Aufbewahrungsstellung außenseitig am Basisgehäuse (2) in einer Sicherungsstellung für die zumindest eine Antriebsbaugruppe (8) gehalten ist, und der Sicherungsstift (16) in der Sicherungsstellung innerhalb des zumindest einen Haltearms (13) im axialen Zentrum der Sicherungsbaugruppe (9) angeordnet ist und den zumindest einen Haltearm (13) der Sicherungsbaugruppe (9) in radialer Richtung bezüglich der Längsachse (11) an einer radialen Verstellung nach innen in Richtung auf die Längsachse (11) ortsfest positioniert hält, und der zumindest eine Haltearm (13) ein Verbindungsglied zur Antriebsbaugruppe (8) bildet,
- wobei zwischen der Sicherungskappe (12) und dem Basisgehäuse (2) eine Führungsanordnung (17) mit zumindest einem ersten Führungselement (19) und mit zumindest einem damit zusammenwirkenden zweiten Führungselement (20) vorgesehen ist,
- wobei die Karpule (6), die Nadelanordnung (7), die zumindest eine Antriebsbaugruppe (8) sowie die Sicherungsbaugruppe (9) zumindest bereichsweise im Basisgehäuse (2) aufgenommen oder angeordnet sind, und die Sicherungsbaugruppe (9) sowie die Antriebsbaugruppe (8) in jenem Teil des Basisgehäuses (2) angeordnet sind, welcher das proximale Ende (4) ausbildet,
- wobei das zumindest eine erste Führungselement (19) durch einen Fortsatz gebildet ist, welcher an einer Innenfläche (21) des Kappenmantels (14) angeordnet oder ausgebildet ist und dieser in Richtung auf die Längsachse (11) vorragt,
- wobei das zumindest eine zweite Führungselement (20) durch eine vertieft im Basisgehäuse (2) ausgebildete Führungsbahn (22) gebildet ist, welche am Basisgehäuse (2) im Bereich des proximalen Endes (4) angeordnet oder ausgebildet ist, oder wobei das zumindest eine zweite Führungselement (20) durch zumindest eine im Bereich des proximalen Endes (4) des Basisgehäuses (2) angeordnete oder ausgebildete, nutförmige Vertiefung und/oder einen Fortsatz gebildet ist,
- wobei die Sicherungskappe (12) ausgehend von ihrer Sicherungsstellung in Axialrichtung sowie auf die vom distalen Ende (3) abgewendete Seite in eine Freigabestellung verstellbar ist, und
- wobei das zumindest eine erste Führungselement (19) und das zumindest eine zweite Führungselement (20) einen in Axialrichtung begrenzten Verstellweg (18) der Sicherungskappe (12) zwischen deren Sicherungsstellung und deren Freigabestellung festlegen, wodurch die Sicherungskappe (12) in deren Freigabestellung noch am Basisgehäuse (2) angeordnet und gehalten ist, und dabei weiters vom Sicherungsstift (16) der zumindest eine Haltearm (13) für die Auslösung und Aktivierung der Antriebsbaugruppe (8) freigegeben ist, **dadurch gekennzeichnet,**
- **dass** die Sicherungsbaugruppe (9) weiters eine Betätigungskappe (32) umfasst, welche lösbar an der Sicherungskappe (12) gehalten ist,
- **dass** die Betätigungskappe (32) die Sicherungskappe (12) im Bereich ihres Kappenmantels (14) übergreift, und
- **dass** zwischen der Betätigungskappe (32) und der Sicherungskappe (12) miteinander in Eingriff stehende Rastmittel (33) vorgesehen sind.

2. Injektionsvorrichtung (1) nach Anspruch 1, **dadurch gekennzeichnet, dass** die Führungsbahn (22) in deren Längsverlauf bezüglich einer in senkrechter Richtung zur Längsachse (11) ausgerichteten Ebene eine unterschiedliche Steigung mit zueinander unterschiedlichen Steigungswinkeln aufweist.

3. Injektionsvorrichtung (1) nach Anspruch 1 oder 2, **dadurch gekennzeichnet, dass** der Steigungswinkel der Führungsbahn (22) im Bereich der Sicherungsstellung und/oder der Freigabestellung flacher ausgebildet ist als in jenem sich zwischen der Sicherungsstellung und der Freigabestellung erstreckenden Zwischenabschnitt derselben.

4. Injektionsvorrichtung (1) nach einem der vorhergehenden Ansprüche, **dadurch gekennzeichnet, dass** die Sicherungsbaugruppe (9) weiters eine Anzeigeeinheit (23) umfasst, welche Anzeigeeinheit (23) zumindest eine der beiden Stellungen der Sicherungskappe (12) relativ bezüglich des Basisgehäuses (2) anzeigt.

5. Injektionsvorrichtung (1) nach Anspruch 4, **dadurch gekennzeichnet, dass** die Anzeigeeinheit (23) zumindest ein erstes Anzeigemittel (24) umfasst, welches im Bereich der Sicherungskappe (12) angeordnet oder ausgebildet ist.

6. Injektionsvorrichtung (1) nach Anspruch 5, **dadurch gekennzeichnet, dass** das zumindest eine erste Anzeigemittel (24) durch einen Durchbruch und/oder einen von der Kappenwand (15) distanziert angeordneten Kappenrand des Kappenmantels (14) gebildet ist.

7. Injektionsvorrichtung (1) nach einem der Ansprüche 4 bis 6, **dadurch gekennzeichnet, dass** die Anzeigeeinheit (23) zumindest ein zweites Anzeigemittel (25) umfasst, welches im Bereich des Basisgehäuses (2) angeordnet oder ausgebildet ist.

8. Injektionsvorrichtung (1) nach Anspruch 4, **dadurch gekennzeichnet, dass** die Anzeigeeinheit (23) weiters ein Anzeigeträgerelement (27) umfasst, welches Anzeigeträgerelement (27) innerhalb des Kappenmantels (14) sowie unmittelbar benachbart zur Kappenwand (15) angeordnet ist, und das Anzeigeträgerelement (27) am Basisgehäuse (2) in Axialrichtung verstellbar geführt ist und weiters die Sicherungskappe (12) relativ bezüglich des Anzeigeträgerelements (27) um die Längsachse (11) verschwenkbar ist und weiters das Anzeigeträgerelement (27) an der Sicherungskappe (12) relativ bezüglich dieser in Richtung der Längsachse (11) positioniert gehalten ist.

9. Injektionsvorrichtung (1) nach Anspruch 8, **dadurch gekennzeichnet, dass** das Anzeigeträgerelement (27) einen scheibenförmig ausgebildeten Grundkörper (28) mit einer in dessen Zentrum angeordneten Durchbrechung (29) sowie zumindest einen in Axialrichtung davon abstehenden Führungssteg (30) umfasst.

10. Injektionsvorrichtung (1) nach Anspruch 9, **dadurch gekennzeichnet, dass** der zumindest eine Führungssteg (30) im äußeren Umfangsbereich des scheibenförmig ausgebildeten Grundkörpers (28) angeordnet ist.

11. Injektionsvorrichtung (1) nach Anspruch 9 oder 10, **dadurch gekennzeichnet, dass** zwischen dem zumindest einen Führungssteg (30) und dem Basisgehäuse (2) eine Längsführungsanordnung (31) angeordnet oder ausgebildet ist.

12. Injektionsvorrichtung (1) nach einem der Ansprüche 4 oder 8 bis 11, **dadurch gekennzeichnet, dass** die Anzeigeeinheit (23) zumindest ein erstes Anzeigemittel (24) umfasst, welches als Durchbruch in der Kappenwand (15) der Sicherungskappe (12) ausgebildet ist.

13. Injektionsvorrichtung (1) nach einem der Ansprüche 8 bis 12, **dadurch gekennzeichnet, dass** die Anzeigeeinheit (23) zumindest ein zweites Anzeigemittel (25) umfasst, welches am Anzeigeträgerelement (27) angeordnet ist.

14. Injektionsvorrichtung (1) nach Anspruch 7 oder 13, **dadurch gekennzeichnet, dass** das zumindest eine zweite Anzeigemittel (25) aus der Gruppe von Symbol, Farbkennung, Markierungsring, nutförmige Vertiefung (26), Bild, Blindenschrift, Brailleschrift ausgewählt ist.

15. Injektionsvorrichtung (1) nach Anspruch 1, **dadurch gekennzeichnet, dass** an der Sicherungskappe (12) als Rastmittel (33) zumindest ein an einem Federarm (34) angeordneter Rastfortsatz (35) und an der Betätigungskappe (32) als weiteres Rastmittel (33) zumindest eine den Rastfortsatz (35) aufnehmende Rastausnehmung (36) vorgesehen sind.

16. Injektionsvorrichtung (1) nach Anspruch 1, **dadurch gekennzeichnet, dass** die miteinander in Eingriff stehenden Rastmittel (33) im Bereich des Kappenmantels (14) vorgesehen sind.

17. Injektionsvorrichtung (1) nach Anspruch 1, **dadurch gekennzeichnet, dass** die Betätigungskappe (32) die Sicherungskappe (12) vollständig in sich aufnimmt.

## Claims

1. An injection device (1) designed as an auto-injector for dispensing an injection fluid, wherein the injection device (1) is designed to be displaceable from a storage position into an injection position, comprising
- a sleeve-shaped base housing (2), which base housing (2) has a distal end (3) which can be turned towards a patient and a proximal end (4) turned away therefrom, between which ends (3, 4) a longitudinal axis (11) extends,
- a carpule (6) containing the injection fluid to be dispensed during the injection procedure,
- a needle arrangement (7), which is disposed in front of the carpule (6) in the section of the distal end (3) in the storage position of the injection device (1),
- at least one drive assembly (8), which drive assembly (8) comprises a drive means for automatically dispensing the injection fluid to be dispensed during the injection procedure,
- a securing assembly (9), which securing assembly (9) secures the at least one drive assembly (8) in its position with respect to the base housing (2) until activated for the injection procedure,
- wherein the securing assembly (9) comprises a securing cap (12) and at least one retaining arm (13), wherein the securing cap (12) comprises a cap jacket (14), a cap wall (15) and a securing pin (16) which is disposed inside the cap jacket (14) and in a center on the cap wall (15) and extends in the direction of the longitudinal axis (11),
- and wherein in the storage position on the outside of the base housing (2), the securing cap (12) is retained in a securing position for the at least one drive assembly (8), and in the securing position, the securing pin (16) is disposed inside the at least one retaining arm (13) at the axial center of the securing assembly (9), and holds the at least one retaining arm (13) of the securing assembly (9) in a stationary position in the radial direction relative to the longitudinal axis (11) preventing a radial movement inwards in the direction towards the longitudinal axis (11), and the at least one retaining arm (13) forms a connecting member to the drive assembly (8),
- wherein a guide arrangement (17) having at least one first guide element (19) and at least one second guide element (20) cooperating therewith is provided between the securing cap (12) and the base housing (2),
- wherein the carpule (6), the needle arrangement (7), the at least one drive assembly (8) and the securing assembly (9) are accommodated or disposed in the base housing (2) in at least certain regions, and the securing assembly (9) and the drive assembly (8) are disposed in that part of the base housing (2) which constitutes the proximal end (4),
- wherein the at least one first guide element (19) is formed by a projection which is disposed or arranged on an internal face (21) of the cap jacket (14) and projects out from said internal face (21) in the direction towards the longitudinal axis (11),
- wherein the at least one second guide element (20) is formed by a guideway (22), which is recessed in the base housing (2), which guideway (22) is arranged or disposed on the base housing (2) in the region of the proximal end (4), or wherein the at least one second guide element (20) is formed by a groove-shaped recess and/or projection disposed or arranged in the region of the proximal end (4) of the base housing (2),
- wherein the securing cap (12) is displaceable from its securing position in the axial direction and towards the end facing away from the distal end (3) into a releasing position,
- wherein the at least one first guide element (19) and the at least one second guide element (20) define an adjustment path (18) of the securing cap (12) delimited in the axial direction between its securing position and its releasing position, whereby the securing cap (12) is still disposed and retained on the base housing (2) in its releasing position, and the at least one retaining arm (13) for triggering and activating the drive assembly (8) is also released by the securing pin (16) at the same time, **characterized in that**
- the securing assembly (9) further comprises an actuation cap (32) which is detachably retained on the securing cap (12),
- the actuation cap (32) overlaps the securing cap (12) in the region of its cap jacket (14), and
- mutually engaging catch means (33) are provided between the actuation cap (32) and the securing cap (12).

2. The injection device (1) according to claim 1, **characterized in that** the guideway (22) has a different pitch with pitch angles different from one another in its longitudinal extension relative to a plane oriented in the direction perpendicular to the longitudinal axis (11).

3. The injection device (1) according to claim 1 or 2, **characterized in that** the pitch angle of the guideway (22) is designed to be flatter in the region of the securing position and/or the releasing position than in the intermediate section thereof extending between the securing position and the releasing position.

4. The injection device (1) according to one of the preceding claims, **characterized in that** the securing assembly (9) further comprises a display unit (23), which display unit (23) displays at least one of the two positions of the securing cap (12) relative to the base housing (2).

5. The injection device (1) according to claim 4, **characterized in that** the display unit (23) comprises at least one first display means (24) which is disposed or arranged in the region of the securing cap (12).

6. The injection device (1) according to claim 5, **characterized in that** the at least one first display means (24) is formed by an orifice and/or a cap rim of the cap jacket (14) disposed at a distance from the cap wall (15).

7. The injection device (1) according to one of claims 4 to 6, **characterized in that** the display unit (23) comprises at least one second display means (25) which is preferably disposed or arranged in the region of the base housing (2).

8. The injection device (1) according to claim 4, **characterized in that** the display unit (23) further comprises a display mounting element (27), which display mounting element (27) is disposed inside the cap jacket (14) and directly adjacent to the cap wall (15), and the display mounting element (27) is guided on the base housing (2) so as to be displaceable in the axial direction, and the securing cap (12) can also be pivoted relative to the display mounting element (27) about the longitudinal axis (11), and the display mounting element (27) is also held in position on the securing cap (12) relative thereto in the direction of the longitudinal axis (11).

9. The injection device (1) according to claim 8, **characterized in that** the display mounting element (27) comprises a disk-shaped base body (28) with an orifice (29) disposed at its center and at least one guide web (30) protruding therefrom in the axial direction.

10. The injection device (1) according to claim 9, **characterized in that** the at least one guide web (30) is disposed in the outer circumferential region of the disk-shaped base body (28).

11. The injection device (1) according to claim 9 or 10, **characterized in that** a longitudinal guide arrangement (31) is disposed or arranged between the at least one guide web (30) and the base housing (2).

12. The injection device (1) according to one of claims 4 or 8 to 11, **characterized in that** the display unit (23) comprises at least one first display means (24) which is formed as an orifice in the cap wall (15) of the securing cap (12).

13. The injection device (1) according to one of claims 8 to 12, **characterized in that** the display unit (23) comprises at least one second display means (25) which is disposed on the display mounting element (27).

14. The injection device (1) according to claim 7 or 13, **characterized in that** the at least one second display means (25) is selected from the group comprising a symbol, color coding, marking ring, groove-shaped recess (26), image, embossed print, braille.

15. The injection device (1) according to claim 1, **characterized in that** on the securing cap (12), at least one catch projection (35) disposed on a spring arm (34) is provided as a catch means (33), and on the actuation cap (32), at least one catch recess (36) accommodating the catch projection (35) is provided as a further catch means (33).

16. The injection device (1) according to claim 1, **characterized in that** the mutually engaging catch means (33) are provided in the region of the cap jacket (14).

17. The injection device (1) according to claim 1, **characterized in that** the actuation cap (32) fully accommodates the securing cap (12) within itself.

## Revendications

1. Dispositif d'injection (1), qui est constitué en tant qu'auto-injecteur pour la délivrance d'un liquide d'injection, le dispositif d'injection (1) étant constitué de façon déplaçable à partir d'une position de stockage vers une position d'injection, avec
- un boîtier de base (2) constitué en forme de gaine, lequel boîtier de base (2) comporte une extrémité distale (3) pouvant être tournée vers un patient et une extrémité proximale (4) qui en est éloignée, un axe longitudinal (11) s'étendant entre ces extrémités (3, 4),
- une ampoule (6) avec le liquide d'injection qui y est contenu et qui est destiné à être délivré lors du processus d'injection,
- un ensemble d'aiguille (7) qui est disposé en amont de l'ampoule (6) dans la position de stockage du dispositif d'injection (1) dans le tronçon de l'extrémité distale (3),
- au moins un module d'entraînement (8), lequel module d'entraînement (8) comprend un moyen d'entraînement destiné à la délivrance automatique du liquide d'injection destiné à délivrer lors du processus d'injection,
- un module de blocage (9), lequel module de blocage (9) immobilise dans sa position par rapport au boîtier de base (2) l'au moins un module d'entraînement (8) jusqu'à son activation pour le processus d'injection,
- le module de blocage (9) comprenant un capuchon de blocage (12) ainsi qu'au moins un bras de support (13), le capuchon de blocage (12) comprenant une enveloppe de capuchon (14), une paroi de capuchon (15) ainsi qu'une broche de blocage (16) disposée à l'intérieur de l'enveloppe de capuchon (14) ainsi que dans un centre sur la paroi de capuchon (15) et s'étendant en direction de l'axe longitudinal (11),
- et le capuchon de blocage (12) étant, dans la position de stockage, retenu côté extérieur sur le boîtier de base (2) dans une position de blocage pour l'au moins un module d'entraînement (8), et la broche de blocage (16) étant, dans la position de blocage, disposée à l'intérieur de l'au moins un bras de support (13) dans le centre axial du module de blocage (9) et retenant, positionné de façon fixe, l'au moins un bras de support (13) du module de blocage (9) dans la direction radiale par rapport à l'axe longitudinal (11) au niveau d'un déplacement radial vers l'intérieur en direction de l'axe longitudinal (11), et l'au moins un bras de support (13) formant un organe de raccordement au module d'entraînement (8),
- un ensemble de guidage (17), avec au moins un premier élément de guidage (19) et avec au moins un deuxième élément de guidage (20) coopérant avec lui, étant prévu entre le capuchon de blocage (12) et le boîtier de base (2),
- l'ampoule (6), l'ensemble d'aiguille (7), l'au moins un module d'entraînement (8) ainsi que le module de blocage (9) étant logés ou disposés au moins par endroits dans le boîtier de base (2), et le module de blocage (9) ainsi que le module d'entraînement (8) étant disposés dans la partie du boîtier de base (2) qui constitue l'extrémité proximale (4),
- l'au moins un premier élément de guidage (19) étant formé par un prolongement qui est disposé ou constitué sur une surface intérieure (21) de l'enveloppe de capuchon (14) et celui-ci dépasse en direction de l'axe longitudinal (11),
- l'au moins un deuxième élément de guidage (20) étant formé par une voie de guidage (22), constituée de façon creusée dans le boîtier de base (2), qui est disposée ou constituée sur le boîtier de base (2) dans la zone de l'extrémité proximale (4), ou l'au moins un deuxième élément de guidage (20) étant formé par au moins un creux en forme de rainure, disposé ou constitué dans la zone de l'extrémité proximale (4) du boîtier de base (2), ou par un prolongement,
- le capuchon de blocage (12) pouvant, à partir de sa position de blocage, être déplacé dans la direction axiale ainsi que vers le côté éloigné de l'extrémité distale (3) dans une position de libération, et
- l'au moins un premier élément de guidage (19) et l'au moins un deuxième élément de guidage (20) immobilisant une course de déplacement (18), limitée dans la direction axiale, du capuchon de blocage (12) entre sa position de blocage et sa position de libération, ce qui fait que le capuchon de blocage (12) est disposé et retenu dans sa position de libération encore sur le boîtier de base (2), et à cette occasion l'au moins un bras de support (13) est en outre libéré de la broche de blocage (16) pour le déclenchement et l'activation du module d'entraînement (8),
**caractérisé en ce que**
- le module de blocage (9) comprend en outre un capuchon d'actionnement (32) qui est retenu sur le capuchon de blocage (12) de façon détachable,
- le capuchon d'actionnement (32) recouvre le capuchon de blocage (12) dans la zone de son enveloppe de capuchon (14), et
- des moyens d'encliquetage (33) engagés les uns avec les autres sont prévus entre le capuchon d'actionnement (32) et le capuchon de blocage (12).

2. Dispositif d'injection (1) selon la revendication 1, **caractérisé en ce que**, dans son tracé longitudinal, la voie de guidage (22) présente, par rapport à un plan orienté dans une direction perpendiculaire par rapport à l'axe longitudinal (11), une pente différente avec des angles de pente différents les uns des autres.

3. Dispositif d'injection (1) selon la revendication 1 ou 2, **caractérisé en ce que**, dans la zone de la position de blocage et/ou de la position de libération, l'angle de pente de la voie de guidage (22) est constitué de façon plus plate que dans le tronçon intermédiaire de cette voie qui s'étend entre la position de blocage et la position de libération.

4. Dispositif d'injection (1) selon l'une des revendications précédentes, **caractérisé en ce que** le module de blocage (9) comprend en outre une unité d'affichage (23), laquelle unité d'affichage (23) indique au moins une des deux positions du capuchon de blocage (12) de façon relative par rapport au boîtier de base (2).

5. Dispositif d'injection (1) selon la revendication 4, **caractérisé en ce que** l'unité d'affichage (23) comprend au moins un premier moyen d'affichage (24) qui est disposé ou constitué dans la zone du capuchon de blocage (12).

6. Dispositif d'injection (1) selon la revendication 5, **caractérisé en ce que** l'au moins un premier moyen d'affichage (24) est formé par un passage ou un bord de capuchon de l'enveloppe de capuchon (14) disposé à distance de la paroi de capuchon (15).

7. Dispositif d'injection (1) selon l'une des revendications 4 à 6, **caractérisé en ce que** l'unité d'affichage (23) comprend un deuxième moyen d'affichage (25), qui est disposé ou constitué dans la zone du boîtier de base (2).

8. Dispositif d'injection (1) selon la revendication 4, **caractérisé en ce que** l'unité d'affichage (23) comprend en outre un élément de support d'affichage (27), lequel élément de support d'affichage (27) est disposé à l'intérieur de l'enveloppe de capuchon (14) ainsi que directement au voisinage de la paroi de capuchon (15), et l'élément de support d'affichage (27) est guidé de façon déplaçable sur le boîtier de base (2) dans la direction axiale, et en outre le capuchon de blocage (12) est disposé de façon pivotante autour de l'axe longitudinal (11) de façon relative par rapport à l'élément de support d'affichage (27), et en outre l'élément de support d'affichage (27) est retenu en étant positionné dans la direction de l'axe longitudinal (11) sur le capuchon de blocage (12) de façon relative par rapport à ce capuchon.

9. Dispositif d'injection (1) selon la revendication 8, **caractérisé en ce que** l'élément de support d'affichage (27) comprend un corps de base (28) constitué en forme de disque avec une traversée (29) disposée dans son centre, ainsi qu'au moins une nervure de guidage (30) qui s'en détache dans la direction axiale.

10. Dispositif d'injection (1) selon la revendication 9, **caractérisé en ce que** l'au moins une nervure de guidage (30) est disposée dans la zone périphérique extérieure du corps de base (28) constitué en forme de disque.

11. Dispositif d'injection (1) selon la revendication 9 ou 10, **caractérisé en ce qu'**un ensemble de guidage longitudinal (31) est disposé ou constitué entre l'au moins une nervure de guidage (30) et le boîtier de base (2).

12. Dispositif d'injection (1) selon l'une des revendications 4 ou 8 à 11, **caractérisé en ce que** l'unité d'affichage (23) comprend au moins un premier moyen d'affichage (24) qui est constitué en tant que passage dans la paroi de capuchon (15) du capuchon de blocage (12).

13. Dispositif d'injection (1) selon l'une des revendications 8 à 12, **caractérisé en ce que** l'unité d'affichage (23) comprend au moins un deuxième moyen d'affichage (25) qui est disposé sur l'élément de support d'affichage (27).

14. Dispositif d'injection (1) selon la revendication 7 ou 13, **caractérisé en ce que** l'au moins un deuxième moyen d'affichage (25) est sélectionné dans le groupe de symbole, code couleur, anneau de repérage, creux (26) en forme de rainure, image, impression en relief à l'usage des aveugles, braille.

15. Dispositif d'injection (1) selon la revendication 1, **caractérisé en ce que**, sur le capuchon de blocage (12), en tant que moyen d'encliquetage (33), il est prévu au moins un prolongement d'encliquetage (35) disposé sur un bras de ressort (34) et, sur le capuchon d'actionnement (32), en tant que moyen d'encliquetage (33) supplémentaire, il est prévu au moins un creux d'encliquetage (36) recevant le prolongement d'encliquetage (35).

16. Dispositif d'injection (1) selon la revendication 1, **caractérisé en ce que** les moyens d'encliquetage (33) engagés les uns avec les autres sont prévus dans la zone de l'enveloppe de capuchon (14).

17. Dispositif d'injection (1) selon la revendication 1, **caractérisé en ce que** le capuchon d'actionnement (32) reçoit en lui-même complètement le capuchon de blocage (12).
